# EUROPEAN PATENT APPLICATION

(11) **EP 0 918 090 A2**
(43) Date of publication of application: **26.05.1999**
(21) Application number: 98308980.6
(22) Date of filing: 03.11.1998
(51) Int. Cl.: C12N 9/02, C12P 21/00, C12P 7/02

(54) **Ketoreductase gene and protein from yeast**

(30) Priority: 04.11.1997 US 64195 P
(71) Applicant: ELI LILLY AND COMPANY, Indianapolis, Indiana 46285 (US)
(72) Inventor: Costello, Colleen Angela, Lawrenceville, New Jersey 08648 (US); Hershberger, Charles Lee, Greenfield, Indiana 46140 (US); Menke, Michael Andrew, Indianapolis, Indiana 46219 (US); Zmijewski, Milton Joseph, Jr., Carmel, Indiana 46033 (US)
(74) Representative: Denholm, Anna Marie

(57) **Abstract**

The invention provides a cloned ketoreductase gene, vectors for expressing same, recombinant host cells that express said vector-borne gene, and a method for stereospecifically reducing a ketone using a recombinant ketoreductase, or a recombinant host cell that expresses a cloned ketoreductase gene.

### Annex to the description

SEQUENCE LISTING

(1) GENERAL INFORMATION:

(i) APPLICANT: ELI LILLY AND COMPANY
(B) STREET: Lilly Corporate Center
(C) CITY: Indianapolis
(D) STATE: Indiana
(E) COUNTRY: United States of America
(F) ZIP: 46285

(ii) TITLE OF INVENTION: Ketoreductase Gene and Protein From Yeast

(iii) NUMBER OF SEQUENCES: 15

(iv) CORRESPONDENCE ADDRESS:
(A) ADDRESSEE: A. M. Denholm
(B) STREET: Erl Wood Manor
(C) CITY: Windlesham
(D) STATE: Surrey
(E) COUNTRY: United Kingdom
(F) ZIP: GU20 6PH

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(2) INFORMATION FOR SEQ ID NO:1:

(i) SEQUENCE CHARACTERISTICS:
(A) LENGTH: 1270 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(ix) FEATURE:
(A) NAME/KEY: CDS
(B) LOCATION: 164..1177
(D) OTHER INFORMATION: Z.rouxii ketoreductase

(xi) SEQUENCE DESCRIPTION: SEQ ID NO:1:

TGAATGGTTA TTTTAGCAAT TGCTGTGTGA GGCACTGACC TAAAGATGTG TATAAATAGT 60

GGGACTGTGT ACTCATGAGG ATCAATACAT GTATAAACTT ACCATACTTT CACACAAGTC 120

AACTTAGAAT CAATCAATCA ATCAATTAAT CAAGCTATAC AAT ATG ACA AAA GTC 175
Met Thr Lys Val
1

TTC GTA ACA GGT GCC AAC GGA TTC GTT GCT CAA CAC GTC GTT CAT CAA 223
Phe Val Thr Gly Ala Asn Gly Phe Val Ala Gln His Val Val His Gln
5                   10                  15                  20

CTA TTA GAA AAG AAC TAT ACA GTG GTT GGA TCT GTC CGT TCA ACT GAG 271
Leu Leu Glu Lys Asn Tyr Thr Val Val Gly Ser Val Arg Ser Thr Glu
                25                  30                  35

AAA GGT GAT AAA TTA GCT AAA TTG CTA AAC AAT CCA AAA TTT TCA TAT 319
Lys Gly Asp Lys Leu Ala Lys Leu Leu Asn Asn Pro Lys Phe Ser Tyr
            40                  45                  50

GAG ATT ATT AAA GAT ATG GTC AAT TCG AGA GAT GAA TTC GAT AAG GCT 367
Glu Ile Ile Lys Asp Met Val Asn Ser Arg Asp Glu Phe Asp Lys Ala
        55                  60                  65

TTA CAA AAA CAT TCA GAT GTT GAA ATT GTC TTA CAT ACT GCT TCA CCA 415
Leu Gln Lys His Ser Asp Val Glu Ile Val Leu His Thr Ala Ser Pro
    70                  75                  80

GTC TTC CCA GGT GGT ATT AAA GAT GTT GAA AAA GAA ATG ATC CAA CCA 463
Val Phe Pro Gly Gly Ile Lys Asp Val Glu Lys Glu Met Ile Gln Pro
85                  90                  95                  100

GCT GTT AAT GGT ACT AGA AAT GTC TTG TTA TCA ATC AAG GAT AAC TTA 511
Ala Val Asn Gly Thr Arg Asn Val Leu Leu Ser Ile Lys Asp Asn Leu
                105                 110                 115

CCA AAT GTC AAG AGA TTT GTT TAC ACT TCT TCA TTA GCT GCT GTC CGT 559
Pro Asn Val Lys Arg Phe Val Tyr Thr Ser Ser Leu Ala Ala Val Arg
            120                 125                 130

ACT GAA GGT GCT GGT TAT AGT GCA GAC GAA GTT GTC ACC GAA GAT TCT 607
Thr Glu Gly Ala Gly Tyr Ser Ala Asp Glu Val Val Thr Glu Asp Ser
        135                 140                 145

TGG AAC AAT ATT GCA TTG AAA GAT GCC ACC AAG GAT GAA GGT ACA GCT 655
Trp Asn Asn Ile Ala Leu Lys Asp Ala Thr Lys Asp Glu Gly Thr Ala
    150                 155                 160

TAT GAG GCT TCC AAG ACA TAT GGT GAA AAA GAA GTT TGG AAT TTC TTC 703
Tyr Glu Ala Ser Lys Thr Tyr Gly Glu Lys Glu Val Trp Asn Phe Phe
165                 170                 175                 180

GAA AAA ACT AAA AAT GTT AAT TTC GAT TTT GCC ATC ATC AAC CCA GTT 751
Glu Lys Thr Lys Asn Val Asn Phe Asp Phe Ala Ile Ile Asn Pro Val
                185                 190                 195

TAT GTC TTT GGT CCT CAA TTA TTT GAA GAA TAC GTT ACT GAT AAA TTG 799
Tyr Val Phe Gly Pro Gln Leu Phe Glu Glu Tyr Val Thr Asp Lys Leu
            200                 205                 210

AAC TTT TCC AGT GAA ATC ATT AAT AGT ATA ATA AAA GGT GAA AAG AAG 847
Asn Phe Ser Ser Glu Ile Ile Asn Ser Ile Ile Lys Gly Glu Lys Lys
        215                 220                 225

GAA ATT GAA GGT TAT GAA ATT GAT GTT AGA GAT ATT GCA AGA GCT CAT 895
Glu Ile Glu Gly Tyr Glu Ile Asp Val Arg Asp Ile Ala Arg Ala His
    230                 235                 240

ATC TCT GCT GTT GAA AAT CCA GCA ACT ACA CGT CAA AGA TTA ATT CCA 943
Ile Ser Ala Val Glu Asn Pro Ala Thr Thr Arg Gln Arg Leu Ile Pro
245                 250                 255                 260

GCA GTT GCA CCA TAC AAT CAA CAA ACT ATC TTG GAT GTT TTG AAT GAA 991
Ala Val Ala Pro Tyr Asn Gln Gln Thr Ile Leu Asp Val Leu Asn Glu
                265                 270                 275

AAC TTC CCA GAA TTG AAA GGT AAA ATC GAT GTT GGG AAA CCA GGT TCT 1039
Asn Phe Pro Glu Leu Lys Gly Lys Ile Asp Val Gly Lys Pro Gly Ser

            280                 285                 290

CAA AAT GAA TTT ATT AAA AAA TAT TAT AAA TTA GAT AAC TCA AAG ACC 1087
Gln Asn Glu Phe Ile Lys Lys Tyr Tyr Lys Leu Asp Asn Ser Lys Thr
        295                 300                 305

AAA AAA GTT TTA GGT TTT GAA TTC ATT TCC CAA GAG CAA ACA ATC AAA 1135
Lys Lys Val Leu Gly Phe Glu Phe Ile Ser Gln Glu Gln Thr Ile Lys
    310                 315                 320

GAT GCT GCT GCT CAA ATC TTG TCC GTT AAA AAT GGA AAA AAA 1177
Asp Ala Ala Ala Gln Ile Leu Ser Val Lys Asn Gly Lys Lys
325                 330                 335

TAAGTGAACT AGACCTGTCA CTATCAGATT ATTAGAGTTC TGTATAGATT AAAGTGTGAA 1237

AATGTATTAG AATCATAATT TTATAATATG CCT 1270

(2) INFORMATION FOR SEQ ID NO:2:

(i) SEQUENCE CHARACTERISTICS:
(A) LENGTH: 338 amino acids
(B) TYPE: amino acid
(D) TOPOLOGY: linear

(ii) MOLECULE TYPE: protein

(xi) SEQUENCE DESCRIPTION: SEQ ID NO:2:

Met Thr Lys Val Phe Val Thr Gly Ala Asn Gly Phe Val Ala Gln His
1               5                   10                  15

Val Val His Gln Leu Leu Glu Lys Asn Tyr Thr Val Val Gly Ser Val
            20                  25                  30

Arg Ser Thr Glu Lys Gly Asp Lys Leu Ala Lys Leu Leu Asn Asn Pro
        35                  40                  45

Lys Phe Ser Tyr Glu Ile Ile Lys Asp Met Val Asn Ser Arg Asp Glu
    50                  55                  60

Phe Asp Lys Ala Leu Gln Lys His Ser Asp Val Glu Ile Val Leu His
65                  70                  75                  80

Thr Ala Ser Pro Val Phe Pro Gly Gly Ile Lys Asp Val Glu Lys Glu
                85                  90                  95

Met Ile Gln Pro Ala Val Asn Gly Thr Arg Asn Val Leu Leu Ser Ile
            100                 105                 110

Lys Asp Asn Leu Pro Asn Val Lys Arg Phe Val Tyr Thr Ser Ser Leu
        115                 120                 125

Ala Ala Val Arg Thr Glu Gly Ala Gly Tyr Ser Ala Asp Glu Val Val
    130                 135                 140

Thr Glu Asp Ser Trp Asn Asn Ile Ala Leu Lys Asp Ala Thr Lys Asp
145                 150                 155                 160

Glu Gly Thr Ala Tyr Glu Ala Ser Lys Thr Tyr Gly Glu Lys Glu Val
                165                 170                 175

Trp Asn Phe Phe Glu Lys Thr Lys Asn Val Asn Phe Asp Phe Ala Ile
            180                 185                 190

Ile Asn Pro Val Tyr Val Phe Gly Pro Gln Leu Phe Glu Glu Tyr Val
        195                 200                 205

Thr Asp Lys Leu Asn Phe Ser Ser Glu Ile Ile Asn Ser Ile Ile Lys
    210                 215                 220

Gly Glu Lys Lys Glu Ile Glu Gly Tyr Glu Ile Asp Val Arg Asp Ile
225                 230                 235                 240

Ala Arg Ala His Ile Ser Ala Val Glu Asn Pro Ala Thr Thr Arg Gln
                245                 250                 255

Arg Leu Ile Pro Ala Val Ala Pro Tyr Asn Gln Gln Thr Ile Leu Asp
            260                 265                 270

Val Leu Asn Glu Asn Phe Pro Glu Leu Lys Gly Lys Ile Asp Val Gly
        275                 280                 285

Lys Pro Gly Ser Gln Asn Glu Phe Ile Lys Lys Tyr Tyr Lys Leu Asp
    290                 295                 300

Asn Ser Lys Thr Lys Lys Val Leu Gly Phe Glu Phe Ile Ser Gln Glu
305                 310                 315                 320

Gln Thr Ile Lys Asp Ala Ala Ala Gln Ile Leu Ser Val Lys Asn Gly
                325                 330                 335

Lys Lys

(2) INFORMATION FOR SEQ ID NO:3:

(i) SEQUENCE CHARACTERISTICS:
(A) LENGTH: 1271 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:3:

UGAAUGGUUA UUUUAGCAAU UGCUGUGUGA GGCACUGACC UAAAGAUGUG UAUAAAUAGU 60

GGGACUGUGU ACUCAUGAGG AUCAAUACAU GUAUAAACUU ACCAUACUUU CACACAAGUC 120

AACUUAGAAU CAAUCAAUCA AUCAAUUAAU CAAGCUAUAC AAUAUGACAA AAGUCUUCGU 180

AACAGGUGCC AACGGAUUCG UUGCUCAACA CGUCGUUCAU CAACUAUUAG AAAAGAACUA 240

UACAGUGGUU GGAUCUGUCC GUUCAACUGA GAAAGGUGAU AAAUUAGCUA AAUUGCUAAA 300

CAAUCCAAAA UUUUCAUAUG AGAUUAUUAA AGAUAUGGUC AAUUCGAGAG AUGAAUUCGA 360

UAAGGCUUUA CAAAAACAUU CAGAUGUUGA AAUUGUCUUA CAUACUGCUU CACCAGUCUU 420

CCCAGGUGGU AUUAAAGAUG UUGAAAAAGA AAUGAUCCAA CCAGCUGUUA AUGGUACUAG 480

AAAUGUCUUG UUAUCAAUCA AGGAUAACUU ACCAAAUGUC AAGAGAUUUG UUUACACUUC 540

UUCAUUAGCU GCUGUCCGUA CUGAAGGUGC UGGUUAUAGU GCAGACGAAG UUGUCACCGA 600

AGAUUCUUGG AACAAUAUUG CAUUGAAAGA UGCCACCAAG GAUGAAGGUA CAGCUUAUGA 660

GGCUUCCAAG ACAUAUGGUG AAAAAGAAGU UUGGAAUUUC UUCGAAAAAA CUAAAAAUGU 720

UAAUUUCGAU UUUGCCAUCA UCAACCCAGU UUAUGUCUUU GGUCCUCAAU UAUUUGAAGA 780

AUACGUUACU GAUAAAUUGA ACUUUUCCAG UGAAAUCAUU AAUAGUAUAA UAAAAGGUGA 840

AAAGAAGGAA AUUGAAGGUU AUGAAAUUGA UGUUAGAGAU AUUGCAAGAG CUCAUAUCUC 900

UGCUGUUGAA AAUCCAGCAA CUACACGUCA AAGAUUAAUU CCAGCAGUUG CACCAUACAA 960

UCAACAAACU AUCUUGGAUG UUUUGAAUGA AAACUUCCCA GAAUUGAAAG GUAAAAUCGA 1020

UGUUGGGAAA CCAGGUUCUC AAAAUGAAUU UAUUAAAAAA UAUUAUAAAU UAGAUAACUC 1080

AAAGACCAAA AAAGUUUUAG GUUUUGAAUU CAUUUCCCAA GAGCAAACAA UCAAAGAUGC 1140

UGCUGCUCAA AUCUUGUCCG UUAAAAAUGG AAAAAAAUAA GUGAACUAGA CCUGUCACUA 1200

UCAGAUUAUU AGAGUUCUGU AUAGAUUAAA GUGUGAAAAU GUAUUAGAAU CAUAAUUUUA 1260

UAAUUAUGCC U 1271

(2) INFORMATION FOR SEQ ID NO:4:

(i) SEQUENCE CHARACTERISTICS:
(A) LENGTH: 1032 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(ix) FEATURE:
(A) NAME/KEY: CDS
(B) LOCATION: 1..1032
(D) OTHER INFORMATION: S.cerevisiae YDR541c

(xi) SEQUENCE DESCRIPTION: SEQ ID NO:4:

ATG TCT AAT ACA GTT CTA GTT TCT GGC GCT TCA GGT TTT ATT GCC TTG 48
Met Ser Asn Thr Val Leu Val Ser Gly Ala Ser Gly Phe Ile Ala Leu
1               5                   10                  15

CAT ATC CTG TCA CAA TTG TTA AAA CAA GAT TAT AAG GTT ATT GGA ACT 96
His Ile Leu Ser Gln Leu Leu Lys Gln Asp Tyr Lys Val Ile Gly Thr
            20                  25                  30

GTG AGA TCC CAT GAA AAA GAA GCA AAA TTG CTA AGA CAA TTT CAA CAT 144
Val Arg Ser His Glu Lys Glu Ala Lys Leu Leu Arg Gln Phe Gln His
        35                  40                  45

AAC CCT AAT TTA ACT TTA GAA ATT GTT CCG GAC ATT TCT CAT CCA AAT 192
Asn Pro Asn Leu Thr Leu Glu Ile Val Pro Asp Ile Ser His Pro Asn
    50                  55                  60

GCT TTC GAT AAG GTT CTG CAG AAA CGT GGA CGT GAG ATT AGG TAT GTT 240

Ala Phe Asp Lys Val Leu Gln Lys Arg Gly Arg Glu Ile Arg Tyr Val
65                  70                  75                  80

CTA CAC ACG GCC TCT CCT TTT CAT TAT GAT ACT ACC GAA TAT GAA AAA     288
Leu His Thr Ala Ser Pro Phe His Tyr Asp Thr Thr Glu Tyr Glu Lys
                85                  90                  95

GAC TTA TTG ATT CCC GCG TTA GAA GGT ACA AAA AAC ATC CTA AAT TCT     336
Asp Leu Leu Ile Pro Ala Leu Glu Gly Thr Lys Asn Ile Leu Asn Ser
            100                 105                 110

ATC AAG AAA TAT GCA GCA GAC ACT GTA GAG CGT GTT GTT GTG ACT TCT     384
Ile Lys Lys Tyr Ala Ala Asp Thr Val Glu Arg Val Val Val Thr Ser
        115                 120                 125

TCT TGT ACT GCT ATT ATA ACC CTT GCA AAG ATG GAC GAT CCC AGT GTG     432
Ser Cys Thr Ala Ile Ile Thr Leu Ala Lys Met Asp Asp Pro Ser Val
    130                 135                 140

GTT TTT ACA GAA GAG AGT TGG AAC GAA GCA ACC TGG GAA AGC TGT CAA     480
Val Phe Thr Glu Glu Ser Trp Asn Glu Ala Thr Trp Glu Ser Cys Gln
145                 150                 155                 160

ATT GAT GGG ATA AAT GCT TAC TTT GCA TCC AAG AAG TTT GCT GAA AAG     528
Ile Asp Gly Ile Asn Ala Tyr Phe Ala Ser Lys Lys Phe Ala Glu Lys
                165                 170                 175

GCT GCC TGG GAG TTC ACA AAA GAG AAT GAA GAT CAC ATC AAA TTC AAA     576
Ala Ala Trp Glu Phe Thr Lys Glu Asn Glu Asp His Ile Lys Phe Lys
            180                 185                 190

CTA ACA ACA GTC AAC CCT TCT CTT CTT TTT GGT CCT CAA CTT TTC GAT     624
Leu Thr Thr Val Asn Pro Ser Leu Leu Phe Gly Pro Gln Leu Phe Asp
        195                 200                 205

GAA GAT GTG CAT GGC CAT TTG AAT ACT TCT TGC GAA ATG ATC AAT GGC     672
Glu Asp Val His Gly His Leu Asn Thr Ser Cys Glu Met Ile Asn Gly
    210                 215                 220

CTA ATT CAT ACC CCA GTA AAT GCC AGT GTT CCT GAT TTT CAT TCC ATT     720
Leu Ile His Thr Pro Val Asn Ala Ser Val Pro Asp Phe His Ser Ile
225                 230                 235                 240

TTT ATT GAT GTA AGG GAT GTG GCC CTA GCT CAT CTG TAT GCT TTC CAG     768
Phe Ile Asp Val Arg Asp Val Ala Leu Ala His Leu Tyr Ala Phe Gln
                245                 250                 255

AAG GAA AAT ACC GCG GGT AAA AGA TTA GTG GTA ACT AAC GGT AAA TTT     816
Lys Glu Asn Thr Ala Gly Lys Arg Leu Val Val Thr Asn Gly Lys Phe
            260                 265                 270

GGA AAC CAA GAT ATC CTG GAT ATT TTG AAC GAA GAT TTT CCA CAA TTA     864
Gly Asn Gln Asp Ile Leu Asp Ile Leu Asn Glu Asp Phe Pro Gln Leu
        275                 280                 285

AGA GGT CTC ATT CCT TTG GGT AAG CCT GGC ACA GGT GAT CAA GTC ATT     912
Arg Gly Leu Ile Pro Leu Gly Lys Pro Gly Thr Gly Asp Gln Val Ile
    290                 295                 300

GAC CGC GGT TCA ACT ACA GAT AAT AGT GCA ACG AGG AAA ATA CTT GGC     960
Asp Arg Gly Ser Thr Thr Asp Asn Ser Ala Thr Arg Lys Ile Leu Gly
305                 310                 315                 320

TTT GAG TTC AGA AGT TTA CAC GAA AGT GTC CAT GAT ACT GCT GCC CAA    1008
Phe Glu Phe Arg Ser Leu His Glu Ser Val His Asp Thr Ala Ala Gln
                325                 330                 335

ATT TTG AAG AAG GAG AAC AGA TTA                                    1032
Ile Leu Lys Lys Glu Asn Arg Leu
            340

(2) INFORMATION FOR SEQ ID NO:5:

(i) SEQUENCE CHARACTERISTICS:
(A) LENGTH: 344 amino acids
(B) TYPE: amino acid
(D) TOPOLOGY: linear

(ii) MOLECULE TYPE: protein

(xi) SEQUENCE DESCRIPTION: SEQ ID NO:5:

Met Ser Asn Thr Val Leu Val Ser Gly Ala Ser Gly Phe Ile Ala Leu
  1               5                  10                  15

His Ile Leu Ser Gln Leu Leu Lys Gln Asp Tyr Lys Val Ile Gly Thr
             20                  25                  30

Val Arg Ser His Glu Lys Glu Ala Lys Leu Leu Arg Gln Phe Gln His
         35                  40                  45

Asn Pro Asn Leu Thr Leu Glu Ile Val Pro Asp Ile Ser His Pro Asn
     50                  55                  60

Ala Phe Asp Lys Val Leu Gln Lys Arg Gly Arg Glu Ile Arg Tyr Val
 65                  70                  75                  80

Leu His Thr Ala Ser Pro Phe His Tyr Asp Thr Thr Glu Tyr Glu Lys
                 85                  90                  95

Asp Leu Leu Ile Pro Ala Leu Glu Gly Thr Lys Asn Ile Leu Asn Ser
            100                 105                 110

Ile Lys Lys Tyr Ala Ala Asp Thr Val Glu Arg Val Val Val Thr Ser
        115                 120                 125

Ser Cys Thr Ala Ile Ile Thr Leu Ala Lys Met Asp Asp Pro Ser Val
    130                 135                 140

Val Phe Thr Glu Glu Ser Trp Asn Glu Ala Thr Trp Glu Ser Cys Gln
145                 150                 155                 160

Ile Asp Gly Ile Asn Ala Tyr Phe Ala Ser Lys Lys Phe Ala Glu Lys
                165                 170                 175

Ala Ala Trp Glu Phe Thr Lys Glu Asn Glu Asp His Ile Lys Phe Lys
            180                 185                 190

Leu Thr Thr Val Asn Pro Ser Leu Leu Phe Gly Pro Gln Leu Phe Asp
        195                 200                 205

Glu Asp Val His Gly His Leu Asn Thr Ser Cys Glu Met Ile Asn Gly
    210                 215                 220

Leu Ile His Thr Pro Val Asn Ala Ser Val Pro Asp Phe His Ser Ile
225                 230                 235                 240

Phe Ile Asp Val Arg Asp Val Ala Leu Ala His Leu Tyr Ala Phe Gln
                245                 250                 255

Lys Glu Asn Thr Ala Gly Lys Arg Leu Val Val Thr Asn Gly Lys Phe

260                 265                 270

Gly Asn Gln Asp Ile Leu Asp Ile Leu Asn Glu Asp Phe Pro Gln Leu
        275                 280                 285

Arg Gly Leu Ile Pro Leu Gly Lys Pro Gly Thr Gly Asp Gln Val Ile
    290                 295                 300

Asp Arg Gly Ser Thr Thr Asp Asn Ser Ala Thr Arg Lys Ile Leu Gly
305                 310                 315                 320

Phe Glu Phe Arg Ser Leu His Glu Ser Val His Asp Thr Ala Ala Gln
                325                 330                 335

Ile Leu Lys Lys Glu Asn Arg Leu
            340

(2) INFORMATION FOR SEQ ID NO:6:

     (i) SEQUENCE CHARACTERISTICS:
          (A) LENGTH: 1032 base pairs
          (B) TYPE: nucleic acid
          (C) STRANDEDNESS: single
          (D) TOPOLOGY: linear

    (ii) MOLECULE TYPE: mRNA

   (iii) HYPOTHETICAL: NO

    (iv) ANTI-SENSE: NO

    (xi) SEQUENCE DESCRIPTION: SEQ ID NO:6:

AUGUCUAAUA CAGUUCUAGU UUCUGGCGCU UCAGGUUUUA UUGCCUUGCA UAUCCUGUCA      60

CAAUUGUUAA AACAAGAUUA UAAGGUUAUU GGAACUGUGA GAUCCCAUGA AAAAGAAGCA     120

AAAUUGCUAA GACAAUUUCA ACAUAACCCU AAUUUAACUU UAGAAAUUGU UCCGGACAUU     180

UCUCAUCCAA AUGCUUUCGA UAAGGUUCUG CAGAAACGUG GACGUGAGAU UAGGUAUGUU     240

CUACACACGG CCUCUCCUUU UCAUUAUGAU ACUACCGAAU AUGAAAAAGA CUUAUUGAUU     300

CCCGCGUUAG AAGGUACAAA AAACAUCCUA AAUUCUAUCA AGAAAUAUGC AGCAGACACU     360

GUAGAGCGUG UUGUUGUGAC UUCUUCUUGU ACUGCUAUUA UAACCCUUGC AAAGAUGGAC     420

GAUCCCAGUG UGGUUUUUAC AGAAGAGAGU UGGAACGAAG CAACCUGGGA AAGCUGUCAA     480

AUUGAUGGGA UAAAUGCUUA CUUUGCAUCC AAGAAGUUUG CUGAAAAGGC UGCCUGGGAG     540

UUCACAAAAG AGAAUGAAGA UCACAUCAAA UUCAAACUAA CAACAGUCAA CCCUUCUCUU     600

CUUUUUGGUC CUCAACUUUU CGAUGAAGAU GUGCAUGGCC AUUUGAAUAC UUCUUGCGAA     660

AUGAUCAAUG GCCUAAUUCA UACCCCAGUA AAUGCCAGUG UUCCUGAUUU UCAUUCCAUU     720

UUUAUUGAUG UAAGGGAUGU GGCCCUAGCU CAUCUGUAUG CUUUCCAGAA GGAAAAUACC     780

GCGGGUAAAA GAUUAGUGGU AACUAACGGU AAAUUUGGAA ACCAAGAUAU CCUGGAUAUU     840

UUGAACGAAG AUUUUCCACA AUUAAGAGGU CUCAUUCCUU UGGGUAAGCC UGGCACAGGU     900

GAUCAAGUCA UUGACCGCGG UUCAACUACA GAUAAUAGUG CAACGAGGAA AAUACUUGGC 960

UUUGAGUUCA GAAGUUUACA CGAAAGUGUC CAUGAUACUG CUGCCCAAAU UUUGAAGAAG 1020

GAGAACAGAU UA 1032

(2) INFORMATION FOR SEQ ID NO:7:

(i) SEQUENCE CHARACTERISTICS:
(A) LENGTH: 1029 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(ix) FEATURE:
(A) NAME/KEY: CDS
(B) LOCATION: 1..1026
(D) OTHER INFORMATION: S.cerevisiae YOL151W

(xi) SEQUENCE DESCRIPTION: SEQ ID NO:7:

ATG TCA GTT TTC GTT TCA GGT GCT AAC GGG TTC ATT GCC CAA CAC ATT 48
Met Ser Val Phe Val Ser Gly Ala Asn Gly Phe Ile Ala Gln His Ile
1               5                   10                  15

GTC GAT CTC CTG TTG AAG GAA GAC TAT AAG GTC ATC GGT TCT GCC AGA 96
Val Asp Leu Leu Leu Lys Glu Asp Tyr Lys Val Ile Gly Ser Ala Arg
            20                  25                  30

AGT CAA GAA AAG GCC GAG AAT TTA ACG GAG GCC TTT GGT AAC AAC CCA 144
Ser Gln Glu Lys Ala Glu Asn Leu Thr Glu Ala Phe Gly Asn Asn Pro
        35                  40                  45

AAA TTC TCC ATG GAA GTT GTC CCA GAC ATA TCT AAG CTG GAC GCA TTT 192
Lys Phe Ser Met Glu Val Val Pro Asp Ile Ser Lys Leu Asp Ala Phe
    50                  55                  60

GAC CAT GTT TTC CAA AAG CAC GGC AAG GAT ATC AAG ATA GTT CTA CAT 240
Asp His Val Phe Gln Lys His Gly Lys Asp Ile Lys Ile Val Leu His
65                  70                  75                  80

ACG GCC TCT CCA TTC TGC TTT GAT ATC ACT GAC AGT GAA CGC GAT TTA 288
Thr Ala Ser Pro Phe Cys Phe Asp Ile Thr Asp Ser Glu Arg Asp Leu
                85                  90                  95

TTA ATT CCT GCT GTG AAC GGT GTT AAG GGA ATT CTC CAC TCA ATT AAA 336
Leu Ile Pro Ala Val Asn Gly Val Lys Gly Ile Leu His Ser Ile Lys
            100                 105                 110

AAA TAC GCC GCT GAT TCT GTA GAA CGT GTA GTT CTC ACC TCT TCT TAT 384
Lys Tyr Ala Ala Asp Ser Val Glu Arg Val Val Leu Thr Ser Ser Tyr
        115                 120                 125

GCA GCT GTG TTC GAT ATG GCA AAA GAA AAC GAT AAG TCT TTA ACA TTT 432
Ala Ala Val Phe Asp Met Ala Lys Glu Asn Asp Lys Ser Leu Thr Phe
    130                 135                 140

AAC GAA GAA TCC TGG AAC CCA GCT ACC TGG GAG AGT TGC CAA AGT GAC 480
Asn Glu Glu Ser Trp Asn Pro Ala Thr Trp Glu Ser Cys Gln Ser Asp

145                 150                 155                 160

CCA GTT AAC GCC TAC TGT GGT TCT AAG AAG TTT GCT GAA AAA GCA GCT          528
Pro Val Asn Ala Tyr Cys Gly Ser Lys Lys Phe Ala Glu Lys Ala Ala
                165                 170                 175

TGG GAA TTT CTA GAG GAG AAT AGA GAC TCT GTA AAA TTC GAA TTA ACT          576
Trp Glu Phe Leu Glu Glu Asn Arg Asp Ser Val Lys Phe Glu Leu Thr
            180                 185                 190

GCC GTT AAC CCA GTT TAC GTT TTT GGT CCG CAA ATG TTT GAC AAA GAT          624
Ala Val Asn Pro Val Tyr Val Phe Gly Pro Gln Met Phe Asp Lys Asp
        195                 200                 205

GTG AAA AAA CAC TTG AAC ACA TCT TGC GAA CTC GTC AAC AGC TTG ATG          672
Val Lys Lys His Leu Asn Thr Ser Cys Glu Leu Val Asn Ser Leu Met
    210                 215                 220

CAT TTA TCA CCA GAG GAC AAG ATA CCG GAA CTA TTT GGT GGA TAC ATT          720
His Leu Ser Pro Glu Asp Lys Ile Pro Glu Leu Phe Gly Gly Tyr Ile
225                 230                 235                 240

GAT GTT CGT GAT GTT GCA AAG GCT CAT TTA GTT GCC TTC CAA AAG AGG          768
Asp Val Arg Asp Val Ala Lys Ala His Leu Val Ala Phe Gln Lys Arg
                245                 250                 255

GAA ACA ATT GGT CAA AGA CTA ATC GTA TCG GAG GCC AGA TTT ACT ATG          816
Glu Thr Ile Gly Gln Arg Leu Ile Val Ser Glu Ala Arg Phe Thr Met
            260                 265                 270

CAG GAT GTT CTC GAT ATC CTT AAC GAA GAC TTC CCT GTT CTA AAA GGC          864
Gln Asp Val Leu Asp Ile Leu Asn Glu Asp Phe Pro Val Leu Lys Gly
        275                 280                 285

AAT ATT CCA GTG GGG AAA CCA GGT TCT GGT GCT ACC CAT AAC ACC CTT          912
Asn Ile Pro Val Gly Lys Pro Gly Ser Gly Ala Thr His Asn Thr Leu
    290                 295                 300

GGT GCT ACT CTT GAT AAT AAA AAG AGT AAG AAA TTG TTA GGT TTC AAG          960
Gly Ala Thr Leu Asp Asn Lys Lys Ser Lys Lys Leu Leu Gly Phe Lys
305                 310                 315                 320

TTC AGG AAC TTG AAA GAG ACC ATT GAC GAC ACT GCC TCC CAA ATT TTA         1008
Phe Arg Asn Leu Lys Glu Thr Ile Asp Asp Thr Ala Ser Gln Ile Leu
                325                 330                 335

AAA TTT GAG GGC AGA ATA TAA                                             1029
Lys Phe Glu Gly Arg Ile
            340

(2) INFORMATION FOR SEQ ID NO:8:

     (i) SEQUENCE CHARACTERISTICS:
          (A) LENGTH: 342 amino acids
          (B) TYPE: amino acid
          (D) TOPOLOGY: linear

    (ii) MOLECULE TYPE: protein

    (xi) SEQUENCE DESCRIPTION: SEQ ID NO:8:

Met Ser Val Phe Val Ser Gly Ala Asn Gly Phe Ile Ala Gln His Ile
  1               5                  10                  15

Val Asp Leu Leu Leu Lys Glu Asp Tyr Lys Val Ile Gly Ser Ala Arg

20                  25                  30

Ser Gln Glu Lys Ala Glu Asn Leu Thr Glu Ala Phe Gly Asn Asn Pro
        35                  40                  45

Lys Phe Ser Met Glu Val Val Pro Asp Ile Ser Lys Leu Asp Ala Phe
    50                  55                  60

Asp His Val Phe Gln Lys His Gly Lys Asp Ile Lys Ile Val Leu His
65                  70                  75                  80

Thr Ala Ser Pro Phe Cys Phe Asp Ile Thr Asp Ser Glu Arg Asp Leu
                85                  90                  95

Leu Ile Pro Ala Val Asn Gly Val Lys Gly Ile Leu His Ser Ile Lys
            100                 105                 110

Lys Tyr Ala Ala Asp Ser Val Glu Arg Val Val Leu Thr Ser Ser Tyr
        115                 120                 125

Ala Ala Val Phe Asp Met Ala Lys Glu Asn Asp Lys Ser Leu Thr Phe
    130                 135                 140

Asn Glu Glu Ser Trp Asn Pro Ala Thr Trp Glu Ser Cys Gln Ser Asp
145                 150                 155                 160

Pro Val Asn Ala Tyr Cys Gly Ser Lys Lys Phe Ala Glu Lys Ala Ala
                165                 170                 175

Trp Glu Phe Leu Glu Glu Asn Arg Asp Ser Val Lys Phe Glu Leu Thr
            180                 185                 190

Ala Val Asn Pro Val Tyr Val Phe Gly Pro Gln Met Phe Asp Lys Asp
        195                 200                 205

Val Lys Lys His Leu Asn Thr Ser Cys Glu Leu Val Asn Ser Leu Met
    210                 215                 220

His Leu Ser Pro Glu Asp Lys Ile Pro Glu Leu Phe Gly Gly Tyr Ile
225                 230                 235                 240

Asp Val Arg Asp Val Ala Lys Ala His Leu Val Ala Phe Gln Lys Arg
                245                 250                 255

Glu Thr Ile Gly Gln Arg Leu Ile Val Ser Glu Ala Arg Phe Thr Met
            260                 265                 270

Gln Asp Val Leu Asp Ile Leu Asn Glu Asp Phe Pro Val Leu Lys Gly
        275                 280                 285

Asn Ile Pro Val Gly Lys Pro Gly Ser Gly Ala Thr His Asn Thr Leu
    290                 295                 300

Gly Ala Thr Leu Asp Asn Lys Lys Ser Lys Lys Leu Leu Gly Phe Lys
305                 310                 315                 320

Phe Arg Asn Leu Lys Glu Thr Ile Asp Asp Thr Ala Ser Gln Ile Leu
                325                 330                 335

Lys Phe Glu Gly Arg Ile
            340

(2) INFORMATION FOR SEQ ID NO:9:

     (i) SEQUENCE CHARACTERISTICS:
          (A) LENGTH: 1026 base pairs

(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:9:

AUGUCAGUUU UCGUUUCAGG UGCUAACGGG UUCAUUGCCC AACACAUUGU CGAUCUCCUG 60

UUGAAGGAAG ACUAUAAGGU CAUCGGUUCU GCCAGAAGUC AAGAAAAGGC CGAGAAUUUA 120

ACGGAGGCCU UUGGUAACAA CCCAAAAUUC UCCAUGGAAG UUGUCCCAGA CAUAUCUAAG 180

CUGGACGCAU UUGACCAUGU UUUCCAAAAG CACGGCAAGG AUAUCAAGAU AGUUCUACAU 240

ACGGCCUCUC CAUUCUGCUU UGAUAUCACU GACAGUGAAC GCGAUUUAUU AAUUCCUGCU 300

GUGAACGGUG UUAAGGGAAU UCUCCACUCA AUUAAAAAAU ACGCCGCUGA UUCUGUAGAA 360

CGUGUAGUUC UCACCUCUUC UUAUGCAGCU GUGUUCGAUA UGGCAAAAGA AAACGAUAAG 420

UCUUUAACAU UUAACGAAGA AUCCUGGAAC CCAGCUACCU GGGAGAGUUG CCAAAGUGAC 480

CCAGUUAACG CCUACUGUGG UUCUAAGAAG UUUGCUGAAA AAGCAGCUUG GGAAUUUCUA 540

GAGGAGAAUA GAGACUCUGU AAAAUUCGAA UUAACUGCCG UUAACCCAGU UUACGUUUUU 600

GGUCCGCAAA UGUUUGACAA AGAUGUGAAA AAACACUUGA ACACAUCUUG CGAACUCGUC 660

AACAGCUUGA UGCAUUUAUC ACCAGAGGAC AAGAUACCGG AACUAUUUGG UGGAUACAUU 720

GAUGUUCGUG AUGUUGCAAA GGCUCAUUUA GUUGCCUUCC AAAAGAGGGA AACAAUUGGU 780

CAAAGACUAA UCGUAUCGGA GGCCAGAUUU ACUAUGCAGG AUGUUCUCGA UAUCCUUAAC 840

GAAGACUUCC CUGUUCUAAA AGGCAAUAUU CCAGUGGGGA AACCAGGUUC UGGUGCUACC 900

CAUAACACCC UUGGUGCUAC UCUUGAUAAU AAAAAGAGUA AGAAAUUGUU AGGUUUCAAG 960

UUCAGGAACU UGAAAGAGAC CAUUGACGAC ACUGCCUCCC AAAUUUUAAA AUUUGAGGGC 1020

AGAAUA 1026

(2) INFORMATION FOR SEQ ID NO:10:

(i) SEQUENCE CHARACTERISTICS:
(A) LENGTH: 1041 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(ix) FEATURE:
(A) NAME/KEY: CDS
(B) LOCATION: 1..1041
(D) OTHER INFORMATION: S. cerevisiae YGL157W

(xi) SEQUENCE DESCRIPTION: SEQ ID NO:10:

ATG ACT ACT GAT ACC ACT GTT TTC GTT TCT GGC GCA ACC GGT TTC ATT 48
Met Thr Thr Asp Thr Thr Val Phe Val Ser Gly Ala Thr Gly Phe Ile
1               5                   10                  15

GCT CTA CAC ATT ATG AAC GAT CTG TTG AAA GCT GGC TAT ACA GTC ATC 96
Ala Leu His Ile Met Asn Asp Leu Leu Lys Ala Gly Tyr Thr Val Ile
            20                  25                  30

GGC TCA GGT AGA TCT CAA GAA AAA AAT GAT GGC TTG CTC AAA AAA TTT 144
Gly Ser Gly Arg Ser Gln Glu Lys Asn Asp Gly Leu Leu Lys Lys Phe
        35                  40                  45

AAT AAC AAT CCC AAA CTA TCG ATG GAA ATT GTG GAA GAT ATT GCT GCT 192
Asn Asn Asn Pro Lys Leu Ser Met Glu Ile Val Glu Asp Ile Ala Ala
    50                  55                  60

CCA AAC GCC TTT GAT GAA GTT TTC AAA AAA CAT GGT AAG GAA ATT AAG 240
Pro Asn Ala Phe Asp Glu Val Phe Lys Lys His Gly Lys Glu Ile Lys
65                  70                  75                  80

ATT GTG CTA CAC ACT GCC TCC CCA TTC CAT TTT GAA ACT ACC AAT TTT 288
Ile Val Leu His Thr Ala Ser Pro Phe His Phe Glu Thr Thr Asn Phe
                85                  90                  95

GAA AAG GAT TTA CTA ACC CCT GCA GTG AAC GGT ACA AAA TCT ATC TTG 336
Glu Lys Asp Leu Leu Thr Pro Ala Val Asn Gly Thr Lys Ser Ile Leu
            100                 105                 110

GAA GCG ATT AAA AAA TAT GCT GCA GAC ACT GTT GAA AAA GTT ATT GTT 384
Glu Ala Ile Lys Lys Tyr Ala Ala Asp Thr Val Glu Lys Val Ile Val
        115                 120                 125

ACT TCG TCT ACT GCT GCT CTG GTG ACA CCT ACA GAC ATG AAC AAA GGA 432
Thr Ser Ser Thr Ala Ala Leu Val Thr Pro Thr Asp Met Asn Lys Gly
    130                 135                 140

GAT TTG GTG ATC ACG GAG GAG AGT TGG AAT AAG GAT ACA TGG GAC AGT 480
Asp Leu Val Ile Thr Glu Glu Ser Trp Asn Lys Asp Thr Trp Asp Ser
145                 150                 155                 160

TGT CAA GCC AAC GCC GTT GCC GCA TAT TGT GGC TCG AAA AAG TTT GCT 528
Cys Gln Ala Asn Ala Val Ala Ala Tyr Cys Gly Ser Lys Lys Phe Ala
                165                 170                 175

GAA AAA ACT GCT TGG GAA TTT CTT AAA GAA AAC AAG TCT AGT GTC AAA 576
Glu Lys Thr Ala Trp Glu Phe Leu Lys Glu Asn Lys Ser Ser Val Lys
            180                 185                 190

TTC ACA CTA TCC ACT ATC AAT CCG GGA TTC GTT TTT GGT CCT CAA ATG 624
Phe Thr Leu Ser Thr Ile Asn Pro Gly Phe Val Phe Gly Pro Gln Met
        195                 200                 205

TTT GCA GAT TCG CTA AAA CAT GGC ATA AAT ACC TCC TCA GGG ATC GTA 672
Phe Ala Asp Ser Leu Lys His Gly Ile Asn Thr Ser Ser Gly Ile Val
    210                 215                 220

TCT GAG TTA ATT CAT TCC AAG GTA GGT GGA GAA TTT TAT AAT TAC TGT 720
Ser Glu Leu Ile His Ser Lys Val Gly Gly Glu Phe Tyr Asn Tyr Cys
225                 230                 235                 240

GGC CCA TTT ATT GAC GTG CGT GAC GTT TCT AAA GCC CAC CTA GTT GCA     768
Gly Pro Phe Ile Asp Val Arg Asp Val Ser Lys Ala His Leu Val Ala
                245                 250                 255

ATT GAA AAA CCA GAA TGT ACC GGC CAA AGA TTA GTA TTG AGT GAA GGT     816
Ile Glu Lys Pro Glu Cys Thr Gly Gln Arg Leu Val Leu Ser Glu Gly
            260                 265                 270

TTA TTC TGC TGT CAA GAA ATC GTT GAC ATC TTG AAC GAG GAA TTC CCT     864
Leu Phe Cys Cys Gln Glu Ile Val Asp Ile Leu Asn Glu Glu Phe Pro
        275                 280                 285

CAA TTA AAG GGC AAG ATA GCT ACA GGT GAA CCT GCG ACC GGT CCA AGC     912
Gln Leu Lys Gly Lys Ile Ala Thr Gly Glu Pro Ala Thr Gly Pro Ser
    290                 295                 300

TTT TTA GAA AAA AAC TCT TGC AAG TTT GAC AAT TCT AAG ACA AAA AAA     960
Phe Leu Glu Lys Asn Ser Cys Lys Phe Asp Asn Ser Lys Thr Lys Lys
305                 310                 315                 320

CTA CTG GGA TTC CAG TTT TAC AAT TTA AAG GAT TGC ATA GTT GAC ACC    1008
Leu Leu Gly Phe Gln Phe Tyr Asn Leu Lys Asp Cys Ile Val Asp Thr
                325                 330                 335

GCG GCG CAA ATG TTA GAA GTT CAA AAT GAA GCC                        1041
Ala Ala Gln Met Leu Glu Val Gln Asn Glu Ala
            340                 345

(2) INFORMATION FOR SEQ ID NO:11:

        (i) SEQUENCE CHARACTERISTICS:
            (A) LENGTH: 347 amino acids
            (B) TYPE: amino acid
            (D) TOPOLOGY: linear

       (ii) MOLECULE TYPE: protein

       (xi) SEQUENCE DESCRIPTION: SEQ ID NO:11:

Met Thr Thr Asp Thr Thr Val Phe Val Ser Gly Ala Thr Gly Phe Ile
  1               5                  10                  15

Ala Leu His Ile Met Asn Asp Leu Leu Lys Ala Gly Tyr Thr Val Ile
             20                  25                  30

Gly Ser Gly Arg Ser Gln Glu Lys Asn Asp Gly Leu Leu Lys Lys Phe
         35                  40                  45

Asn Asn Asn Pro Lys Leu Ser Met Glu Ile Val Glu Asp Ile Ala Ala
     50                  55                  60

Pro Asn Ala Phe Asp Glu Val Phe Lys Lys His Gly Lys Glu Ile Lys
 65                  70                  75                  80

Ile Val Leu His Thr Ala Ser Pro Phe His Phe Glu Thr Thr Asn Phe
                 85                  90                  95

Glu Lys Asp Leu Leu Thr Pro Ala Val Asn Gly Thr Lys Ser Ile Leu
            100                 105                 110

Glu Ala Ile Lys Lys Tyr Ala Ala Asp Thr Val Glu Lys Val Ile Val
        115                 120                 125

Thr Ser Ser Thr Ala Ala Leu Val Thr Pro Thr Asp Met Asn Lys Gly

130                 135                 140

Asp Leu Val Ile Thr Glu Glu Ser Trp Asn Lys Asp Thr Trp Asp Ser
145                 150                 155                 160

Cys Gln Ala Asn Ala Val Ala Ala Tyr Cys Gly Ser Lys Lys Phe Ala
                165                 170                 175

Glu Lys Thr Ala Trp Glu Phe Leu Lys Glu Asn Lys Ser Ser Val Lys
            180                 185                 190

Phe Thr Leu Ser Thr Ile Asn Pro Gly Phe Val Phe Gly Pro Gln Met
        195                 200                 205

Phe Ala Asp Ser Leu Lys His Gly Ile Asn Thr Ser Ser Gly Ile Val
    210                 215                 220

Ser Glu Leu Ile His Ser Lys Val Gly Gly Glu Phe Tyr Asn Tyr Cys
225                 230                 235                 240

Gly Pro Phe Ile Asp Val Arg Asp Val Ser Lys Ala His Leu Val Ala
                245                 250                 255

Ile Glu Lys Pro Glu Cys Thr Gly Gln Arg Leu Val Leu Ser Glu Gly
            260                 265                 270

Leu Phe Cys Cys Gln Glu Ile Val Asp Ile Leu Asn Glu Glu Phe Pro
        275                 280                 285

Gln Leu Lys Gly Lys Ile Ala Thr Gly Glu Pro Ala Thr Gly Pro Ser
    290                 295                 300

Phe Leu Glu Lys Asn Ser Cys Lys Phe Asp Asn Ser Lys Thr Lys Lys
305                 310                 315                 320

Leu Leu Gly Phe Gln Phe Tyr Asn Leu Lys Asp Cys Ile Val Asp Thr
                325                 330                 335

Ala Ala Gln Met Leu Glu Val Gln Asn Glu Ala
            340                 345

(2) INFORMATION FOR SEQ ID NO:12:

    (i) SEQUENCE CHARACTERISTICS:
        (A) LENGTH: 1041 base pairs
        (B) TYPE: nucleic acid
        (C) STRANDEDNESS: single
        (D) TOPOLOGY: linear

    (ii) MOLECULE TYPE: mRNA

    (iii) HYPOTHETICAL: NO

    (iv) ANTI-SENSE: NO

    (xi) SEQUENCE DESCRIPTION: SEQ ID NO:12:

AUGACUACUG AUACCACUGU UUUCGUUUCU GGCGCAACCG GUUUCAUUGC UCUACACAUU      60

AUGAACGAUC UGUUGAAAGC UGGCUAUACA GUCAUCGGCU CAGGUAGAUC UCAAGAAAAA     120

AAUGAUGGCU UGCUCAAAAA AUUUAAUAAC AAUCCCAAAC UAUCGAUGGA AAUUGUGGAA     180

GAUAUUGCUG CUCCAAACGC CUUUGAUGAA GUUUUCAAAA AACAUGGUAA GGAAAUUAAG 240

AUUGUGCUAC ACACUGCCUC CCCAUUCCAU UUUGAAACUA CCAAUUUUGA AAAGGAUUUA 300

CUAACCCCUG CAGUGAACGG UACAAAAUCU AUCUUGGAAG CGAUUAAAAA AUAUGCUGCA 360

GACACUGUUG AAAAAGUUAU UGUUACUUCG UCUACUGCUG CUCUGGUGAC ACCUACAGAC 420

AUGAACAAAG GAGAUUUGGU GAUCACGGAG GAGAGUUGGA AUAAGGAUAC AUGGGACAGU 480

UGUCAAGCCA ACGCCGUUGC CGCAUAUUGU GGCUCGAAAA AGUUUGCUGA AAAAACUGCU 540

UGGGAAUUUC UUAAAGAAAA CAAGUCUAGU GUCAAAUUCA CACUAUCCAC UAUCAAUCCG 600

GGAUUCGUUU UUGGUCCUCA AAUGUUUGCA GAUUCGCUAA AACAUGGCAU AAAUACCUCC 660

UCAGGGAUCG UAUCUGAGUU AAUUCAUUCC AAGGUAGGUG GAGAAUUUUA UAAUUACUGU 720

GGCCCAUUUA UUGACGUGCG UGACGUUUCU AAAGCCCACC UAGUUGCAAU UGAAAAACCA 780

GAAUGUACCG GCCAAAGAUU AGUAUUGAGU GAAGGUUUAU UCUGCUGUCA AGAAAUCGUU 840

GACAUCUUGA ACGAGGAAUU CCCUCAAUUA AAGGGCAAGA UAGCUACAGG UGAACCUGCG 900

ACCGGUCCAA GCUUUUUAGA AAAAAACUCU UGCAAGUUUG ACAAUUCUAA GACAAAAAAA 960

CUACUGGGAU UCCAGUUUUA CAAUUUAAAG GAUUGCAUAG UUGACACCGC GGCGCAAAUG 1020

UUAGAAGUUC AAAAUGAAGC C 1041

(2) INFORMATION FOR SEQ ID NO:13:

(i) SEQUENCE CHARACTERISTICS:
(A) LENGTH: 1044 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(ix) FEATURE:
(A) NAME/KEY: CDS
(B) LOCATION: 1..1044
(D) OTHER INFORMATION: S. cerevisiae YGL039W

(xi) SEQUENCE DESCRIPTION: SEQ ID NO:13:

ATG ACT ACT GAA AAA ACC GTT GTT TTT GTT TCT GGT GCT ACT GGT TTC 48
Met Thr Thr Glu Lys Thr Val Val Phe Val Ser Gly Ala Thr Gly Phe
1               5                   10                  15

ATT GCT CTA CAC GTA GTG GAC GAT TTA TTA AAA ACT GGT TAC AAG GTC 96
Ile Ala Leu His Val Val Asp Asp Leu Leu Lys Thr Gly Tyr Lys Val
            20                  25                  30

ATC GGT TCG GGT AGG TCC CAA GAA AAG AAT GAT GGA TTG CTG AAA AAA 144
Ile Gly Ser Gly Arg Ser Gln Glu Lys Asn Asp Gly Leu Leu Lys Lys
        35                  40                  45

TTT AAG AGC AAT CCC AAC CTT TCA ATG GAG ATT GTC GAA GAC ATT GCT 192
Phe Lys Ser Asn Pro Asn Leu Ser Met Glu Ile Val Glu Asp Ile Ala

    50                  55                  60

GCT CCA AAC GCT TTT GAC AAA GTT TTT CAA AAG CAC GGC AAA GAG ATC 240
Ala Pro Asn Ala Phe Asp Lys Val Phe Gln Lys His Gly Lys Glu Ile
65                  70                  75                  80

AAG GTT GTC TTG CAC ATA GCT TCT CCG GTT CAC TTC AAC ACC ACT GAT 288
Lys Val Val Leu His Ile Ala Ser Pro Val His Phe Asn Thr Thr Asp
                85                  90                  95

TTC GAA AAG GAT CTG CTA ATT CCT GCT GTG AAT GGT ACC AAG TCC ATT 336
Phe Glu Lys Asp Leu Leu Ile Pro Ala Val Asn Gly Thr Lys Ser Ile
            100                 105                 110

CTA GAA GCA ATC AAA AAT TAT GCC GCA GAC ACA GTC GAA AAA GTC GTT 384
Leu Glu Ala Ile Lys Asn Tyr Ala Ala Asp Thr Val Glu Lys Val Val
        115                 120                 125

ATT ACT TCT TCT GTT GCT GCC CTT GCA TCT CCC GGA GAT ATG AAG GAC 432
Ile Thr Ser Ser Val Ala Ala Leu Ala Ser Pro Gly Asp Met Lys Asp
    130                 135                 140

ACT AGT TTC GTT GTC AAT GAG GAA AGT TGG AAC AAA GAT ACT TGG GAA 480
Thr Ser Phe Val Val Asn Glu Glu Ser Trp Asn Lys Asp Thr Trp Glu
145                 150                 155                 160

AGT TGT CAA GCT AAC GCG GTT TCC GCA TAC TGT GGT TCC AAG AAA TTT 528
Ser Cys Gln Ala Asn Ala Val Ser Ala Tyr Cys Gly Ser Lys Lys Phe
                165                 170                 175

GCT GAA AAA ACT GCT TGG GAT TTT CTC GAG GAA AAC CAA TCA AGC ATC 576
Ala Glu Lys Thr Ala Trp Asp Phe Leu Glu Glu Asn Gln Ser Ser Ile
            180                 185                 190

AAA TTT ACG CTA TCA ACC ATC AAC CCA GGA TTT GTT TTT GGC CCT CAG 624
Lys Phe Thr Leu Ser Thr Ile Asn Pro Gly Phe Val Phe Gly Pro Gln
        195                 200                 205

CTA TTT GCC GAC TCT CTT AGA AAT GGA ATA AAT AGC TCT TCA GCC ATT 672
Leu Phe Ala Asp Ser Leu Arg Asn Gly Ile Asn Ser Ser Ser Ala Ile
    210                 215                 220

ATT GCC AAT TTG GTT AGT TAT AAA TTA GGC GAC AAT TTT TAT AAT TAC 720
Ile Ala Asn Leu Val Ser Tyr Lys Leu Gly Asp Asn Phe Tyr Asn Tyr
225                 230                 235                 240

AGT GGT CCT TTT ATT GAC GTT CGC GAT GTT TCA AAA GCT CAT TTA CTT 768
Ser Gly Pro Phe Ile Asp Val Arg Asp Val Ser Lys Ala His Leu Leu
                245                 250                 255

GCA TTT GAG AAA CCC GAA TGC GCT GGC CAA AGA CTA TTC TTA TGT GAA 816
Ala Phe Glu Lys Pro Glu Cys Ala Gly Gln Arg Leu Phe Leu Cys Glu
            260                 265                 270

GAT ATG TTT TGC TCT CAA GAA GCG CTG GAT ATC TTG AAT GAG GAA TTT 864
Asp Met Phe Cys Ser Gln Glu Ala Leu Asp Ile Leu Asn Glu Glu Phe
        275                 280                 285

CCA CAG TTA AAA GGC AAG ATA GCA ACT GGC GAA CCT GGT AGC GGC TCA 912
Pro Gln Leu Lys Gly Lys Ile Ala Thr Gly Glu Pro Gly Ser Gly Ser
    290                 295                 300

ACC TTT TTG ACA AAA AAC TGC TGC AAG TGC GAC AAC CGC AAA ACC AAA 960
Thr Phe Leu Thr Lys Asn Cys Cys Lys Cys Asp Asn Arg Lys Thr Lys
305                 310                 315                 320

AAT TTA TTA GGA TTC CAA TTT AAT AAG TTC AGA GAT TGC ATT GTC GAT 1008
Asn Leu Leu Gly Phe Gln Phe Asn Lys Phe Arg Asp Cys Ile Val Asp
                325                 330                 335

ACT GCC TCG CAA TTA CTA GAA GTT CAA AGT AAA AGC 1044
Thr Ala Ser Gln Leu Leu Glu Val Gln Ser Lys Ser
            340                 345

(2) INFORMATION FOR SEQ ID NO:14:

(i) SEQUENCE CHARACTERISTICS:
(A) LENGTH: 348 amino acids
(B) TYPE: amino acid
(D) TOPOLOGY: linear

(ii) MOLECULE TYPE: protein

(xi) SEQUENCE DESCRIPTION: SEQ ID NO:14:

Met Thr Thr Glu Lys Thr Val Val Phe Val Ser Gly Ala Thr Gly Phe
1               5                   10                  15

Ile Ala Leu His Val Val Asp Asp Leu Leu Lys Thr Gly Tyr Lys Val
            20                  25                  30

Ile Gly Ser Gly Arg Ser Gln Glu Lys Asn Asp Gly Leu Leu Lys Lys
        35                  40                  45

Phe Lys Ser Asn Pro Asn Leu Ser Met Glu Ile Val Glu Asp Ile Ala
    50                  55                  60

Ala Pro Asn Ala Phe Asp Lys Val Phe Gln Lys His Gly Lys Glu Ile
65                  70                  75                  80

Lys Val Val Leu His Ile Ala Ser Pro Val His Phe Asn Thr Thr Asp
                85                  90                  95

Phe Glu Lys Asp Leu Leu Ile Pro Ala Val Asn Gly Thr Lys Ser Ile
            100                 105                 110

Leu Glu Ala Ile Lys Asn Tyr Ala Ala Asp Thr Val Glu Lys Val Val
        115                 120                 125

Ile Thr Ser Ser Val Ala Ala Leu Ala Ser Pro Gly Asp Met Lys Asp
    130                 135                 140

Thr Ser Phe Val Val Asn Glu Glu Ser Trp Asn Lys Asp Thr Trp Glu
145                 150                 155                 160

Ser Cys Gln Ala Asn Ala Val Ser Ala Tyr Cys Gly Ser Lys Lys Phe
                165                 170                 175

Ala Glu Lys Thr Ala Trp Asp Phe Leu Glu Glu Asn Gln Ser Ser Ile
            180                 185                 190

Lys Phe Thr Leu Ser Thr Ile Asn Pro Gly Phe Val Phe Gly Pro Gln
        195                 200                 205

Leu Phe Ala Asp Ser Leu Arg Asn Gly Ile Asn Ser Ser Ser Ala Ile
    210                 215                 220

Ile Ala Asn Leu Val Ser Tyr Lys Leu Gly Asp Asn Phe Tyr Asn Tyr
225                 230                 235                 240

Ser Gly Pro Phe Ile Asp Val Arg Asp Val Ser Lys Ala His Leu Leu

245                    250                    255

Ala Phe Glu Lys Pro Glu Cys Ala Gly Gln Arg Leu Phe Leu Cys Glu
            260                     265                     270

Asp Met Phe Cys Ser Gln Glu Ala Leu Asp Ile Leu Asn Glu Glu Phe
        275                     280                     285

Pro Gln Leu Lys Gly Lys Ile Ala Thr Gly Glu Pro Gly Ser Gly Ser
    290                     295                     300

Thr Phe Leu Thr Lys Asn Cys Cys Lys Cys Asp Asn Arg Lys Thr Lys
305                     310                     315                     320

Asn Leu Leu Gly Phe Gln Phe Asn Lys Phe Arg Asp Cys Ile Val Asp
                325                     330                     335

Thr Ala Ser Gln Leu Leu Glu Val Gln Ser Lys Ser
            340                     345

(2) INFORMATION FOR SEQ ID NO:15:

(i) SEQUENCE CHARACTERISTICS:
(A) LENGTH: 1044 base pairs
(B) TYPE: nucleic acid
(C) STRANDEDNESS: single
(D) TOPOLOGY: linear

(ii) MOLECULE TYPE: mRNA

(iii) HYPOTHETICAL: NO

(iv) ANTI-SENSE: NO

(xi) SEQUENCE DESCRIPTION: SEQ ID NO:15:

AUGACUACUG AAAAAACCGU UGUUUUUGUU UCUGGUGCUA CUGGUUUCAU UGCUCUACAC     60

GUAGUGGACG AUUUAUUAAA AACUGGUUAC AAGGUCAUCG GUUCGGGUAG GUCCCAAGAA    120

AAGAAUGAUG GAUUGCUGAA AAAAUUUAAG AGCAAUCCCA ACCUUUCAAU GGAGAUUGUC    180

GAAGACAUUG CUGCUCCAAA CGCUUUUGAC AAAGUUUUUC AAAAGCACGG CAAAGAGAUC    240

AAGGUUGUCU UGCACAUAGC UUCUCCGGUU CACUUCAACA CCACUGAUUU CGAAAAGGAU    300

CUGCUAAUUC CUGCUGUGAA UGGUACCAAG UCCAUUCUAG AAGCAAUCAA AAAUUAUGCC    360

GCAGACACAG UCGAAAAAGU CGUUAUUACU UCUUCUGUUG CUGCCCUUGC AUCUCCCGGA    420

GAUAUGAAGG ACACUAGUUU CGUUGUCAAU GAGGAAAGUU GGAACAAAGA UACUUGGGAA    480

AGUUGUCAAG CUAACGCGGU UUCCGCAUAC UGUGGUUCCA AGAAAUUUGC UGAAAAAACU    540

GCUUGGGAUU UUCUCGAGGA AAACCAAUCA AGCAUCAAAU UUACGCUAUC AACCAUCAAC    600

CCAGGAUUUG UUUUUGGCCC UCAGCUAUUU GCCGACUCUC UUAGAAAUGG AAUAAAUAGC    660

UCUUCAGCCA UUAUUGCCAA UUUGGUUAGU UAUAAAUUAG GCGACAAUUU UUAUAAUUAC    720

AGUGGUCCUU UUAUUGACGU UCGCGAUGUU UCAAAAGCUC AUUUACUUGC AUUUGAGAAA    780

CCCGAAUGCG CUGGCCAAAG ACUAUUCUUA UGUGAAGAUA UGUUUUGCUC UCAAGAAGCG    840

CUGGAUAUCU UGAAUGAGGA AUUUCCACAG UUAAAAGGCA AGAUAGCAAC UGGCGAACCU 900
GGUAGCGGCU CAACCUUUUU GACAAAAAAC UGCUGCAAGU GCGACAACCG CAAAACCAAA 960
AAUUUAUUAG GAUUCCAAUU UAAUAAGUUC AGAGAUUGCA UUGUCGAUAC UGCCUCGCAA 1020
UUACUAGAAG UUCAAAGUAA AAGC 1044

## Description

This application claims the benefit of U.S. Provisional Application No. 60/064,195, filed November 4, 1997.

This invention relates to recombinant DNA technology. In particular the invention pertains to the cloning of a ketoreductase gene from *Zygosaccharomyces rouxii,* and the use of recombinant hosts expressing fungal ketoreductase genes in a process for stereospecific reduction of ketones.

2,3 Benzodiazepine derivatives are potent antagonists of the AMPA (α-amino-3-hydroxy-5 methylisoxazole-4-propionic acid) class of receptors in the mammalian central nervous system (*See* I. Tarnawa *et al.* In *Amino Acids: Chemistry, Biology and Medicine,* Eds. Lubec and Rosenthal, Leiden, 1990). These derivative compounds have potentially widespread applications as neuroprotective agents, particularly as anti-convulsants. One series of 2,3 benzodiazepines is considered particularly advantageous for such use, and this series of compounds has the following general formula: 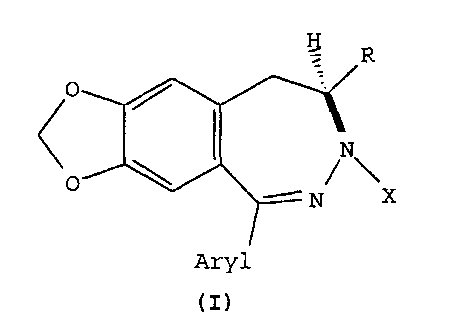
Wherein R is hydrogen or C₁-C₁₀ alkyl; and
X is hydrogen, C₁-C₁₀ alkyl, acyl, aryl, amido or carboxyl, or a substituted derivative thereof.

The clinical potential for these compounds has led to interest in developing more efficient synthetic methods. Biologically-based methods in which a ketoreductase enzyme provides a stereospecific reduction in a whole-cell process using fungal cells have been described in U.S. Patent application serial number 08/413,036.

The present invention provides isolated nucleic acid molecules that encode a ketoreductase enzyme from *Z. rouxii.* The invention also provides the protein product of said nucleic acid, in substantially purified form. Also provided are methods for the formation of chiral alcohols using a purified ketoreductase enzyme, or a recombinant host cell that expresses a fungal ketoreductase gene.

Having the cloned ketoreductase gene enables the production of recombinant ketoreductase protein, and the production of recombinant host cells expressing said protein, wherein said recombinant cells can be used in a stereospecific reduction of ketones.

In one embodiment the present invention relates to an isolated DNA molecule encoding ketoreductase protein, said DNA molecule comprising the nucleotide sequence identified as SEQ ID NO:1.

In another embodiment the present invention relates to a substantially purified ketoreductase protein molecule from *Z. rouxii.*

In another embodiment the present invention relates to a ketoreductase protein molecule from *Z. rouxii,* wherein said protein molecule comprises the sequence identified as SEQ ID NO:2.

In a further embodiment the present invention relates to a ribonucleic acid molecule encoding ketoreductase protein, said ribonucleic acid molecule comprising the sequence identified as SEQ ID NO:3.

In yet another embodiment, the present invention relates to a recombinant DNA vector that incorporates a ketoreductase gene in operable-linkage to gene expression sequences, enabling said gene to be transcribed and translated in a host cell.

In still another embodiment the present invention relates to host cells that have been transformed or transfected with a cloned ketoreductase gene such that said ketoreductase gene is expressed in the host cell.

In a still further embodiment, the present invention relates to a method for producing chiral alcohols using recombinant host cells that express an exogenously introduced ketoreductase gene.

In yet another embodiment, the present invention relates to a method for producing chiral alcohols using recombinant host cells that have been transformed or transfected with a ketoreductase gene from *Z. rouxii, or S. cerevisiae.*

In yet another embodiment, the present invention relates to a method for producing chiral alcohols using a purified fungal ketoreductase.

### Definitions

SEQ ID NO:1 - SEQ ID NO:3 comprises the DNA, protein, and RNA sequences of ketoreductase from *Z. rouxii.*
SEQ ID NO:4- SEQ ID NO:6 comprises the DNA, protein, and RNA sequences of gene YDR541c from *S. cerevisiae.*
SEQ ID NO:7- SEQ ID NO:9 comprises the DNA, protein, and RNA sequences of YOL151w from *S. cerevisiae.*
SEQ ID NO:10- SEQ ID NO:12 comprises the DNA, protein, and RNA sequences of YGL157w from *S. cerevisiae.*
SEQ ID NO:13- SEQ ID NO:15 comprises the DNA, protein, and RNA sequences of YGL039w from *S. cerevisiae.*

The term "fusion protein" denotes a hybrid protein molecule not found in nature comprising a translational fusion or enzymatic fusion in which two or more different proteins or fragments thereof are covalently linked on a single polypeptide chain.

The term "plasmid" refers to an extrachromosomal genetic element. The starting plasmids herein are either commercially available, publicly available on an unrestricted basis, or can be constructed from available plasmids in accordance with published procedures. In addition, equivalent plasmids to those described are known in the art and will be apparent to the ordinarily skilled artisan.

"Recombinant DNA cloning vector" as used herein refers to any autonomously replicating agent, including, but not limited to, plasmids and phages, comprising a DNA molecule to which one or more additional DNA segments can or have been added.

The term "recombinant DNA expression vector" or "expression vector" as used herein refers to any recombinant DNA cloning vector, for example a plasmid or phage, in which a promoter and other regulatory elements are present thereby enabling transcription of an inserted DNA.

The term "vector" as used herein refers to a nucleic acid compound used for introducing exogenous DNA into host cells. A vector comprises a nucleotide sequence which may encode one or more protein molecules. Plasmids, cosmids, viruses, and bacteriophages, in the natural state or which have undergone recombinant engineering, are examples of commonly used vectors.

The terms "complementary" or "complementarity" as used herein refers to the capacity of purine and pyrimidine nucleotides to associate through hydrogen bonding in double stranded nucleic acid molecules. The following base pairs are complementary: guanine and cytosine; adenine and thymine; and adenine and uracil. As used herein "complementary" means that at least one of two hybridizing strands is fully base-paired with the other member of said hybridizing strands, and there are no mismatches. Moreover, at each nucleotide position of said one strand, an "A" is paired with a "T", a "T" is paired with an "A", a "G" is paired with a "C", and a "C" is paired with a "G".

"Isolated nucleic acid compound" refers to any RNA or DNA sequence, however constructed or synthesized, which is locationally distinct from its natural location.

A "primer" is a nucleic acid fragment which functions as an initiating substrate for enzymatic or synthetic elongation of, for example, a nucleic acid molecule.

The term "promoter" refers to a DNA sequence which directs transcription of DNA to RNA. An inducible promoter is one that is regulatable by environmental signals, such as carbon source, heat, metal ions, chemical inducers, etc.; a constitutive promoter generally is expressed at a constant level and is not regulatable.

A "probe" as used herein is a labeled nucleic acid compound which can hybridize wih another nucleic acid compound.

The term "hybridization" as used herein refers to a process in which a single-stranded nucleic acid molecule joins with a complementary strand through nucleotide base pairing. "Selective hybridization" refers to hybridization under conditions of high stringency. The degree of hybridization depends upon, for example, the degree of complementarity, the stringency of hybridization, and the length of hybridizing strands.

"Substantially identical" means a sequence having sufficient homology to hybridize under stringent conditions and/or be at least 90% identical to a sequence disclosed herein.

The term "stringency" relates to nucleic acid hybridization conditions. High stringency conditions disfavor non-homologous base pairing. Low stringency conditions have the opposite effect. Stringency may be altered, for example, by changes in temperature, denaturants, and salt concentration. Typical high stringency conditions comprise hybridizing at 50°C to 65°C in 5X SSPE and 50% formamide, and washing at 50°C to 65°C in 0.5X SSPE; typical low stringency conditions comprise hybridizing at 35°C to 37° in 5X SSPE and 40% to 45% formamide and washing at 42°C in 1X-2X SSPE.

"SSPE" denotes a hybridization and wash solution comprising sodium chloride, sodium phosphate, and EDTA, at pH 7.4. A 20X solution of SSPE is made by dissolving 174 g of NaCl, 27.6 g of NaH₂PO4·H₂O, and 7.4 g of EDTA in 800 ml of H₂O. The pH is adjusted with NaOH and the volume brought to 1 liter.

"SSC" denotes a hybridization and wash solution comprising sodium chloride and sodium citrate at pH 7. A 20X solution of SSC is made by dissolving 175 g of NaCl and 88 g of sodium citrate in 800 ml of H₂O. The volume is brought to 1 liter after adjusting the pH with 10N NaOH.

The ketoreductase gene encodes a novel enzyme that catalyzes an asymmetric reduction of selected ketone substrates (*See* Equation 1 and Table 1).

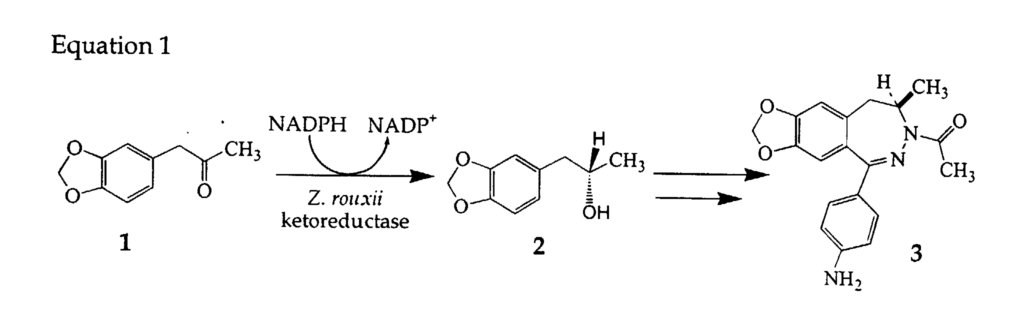

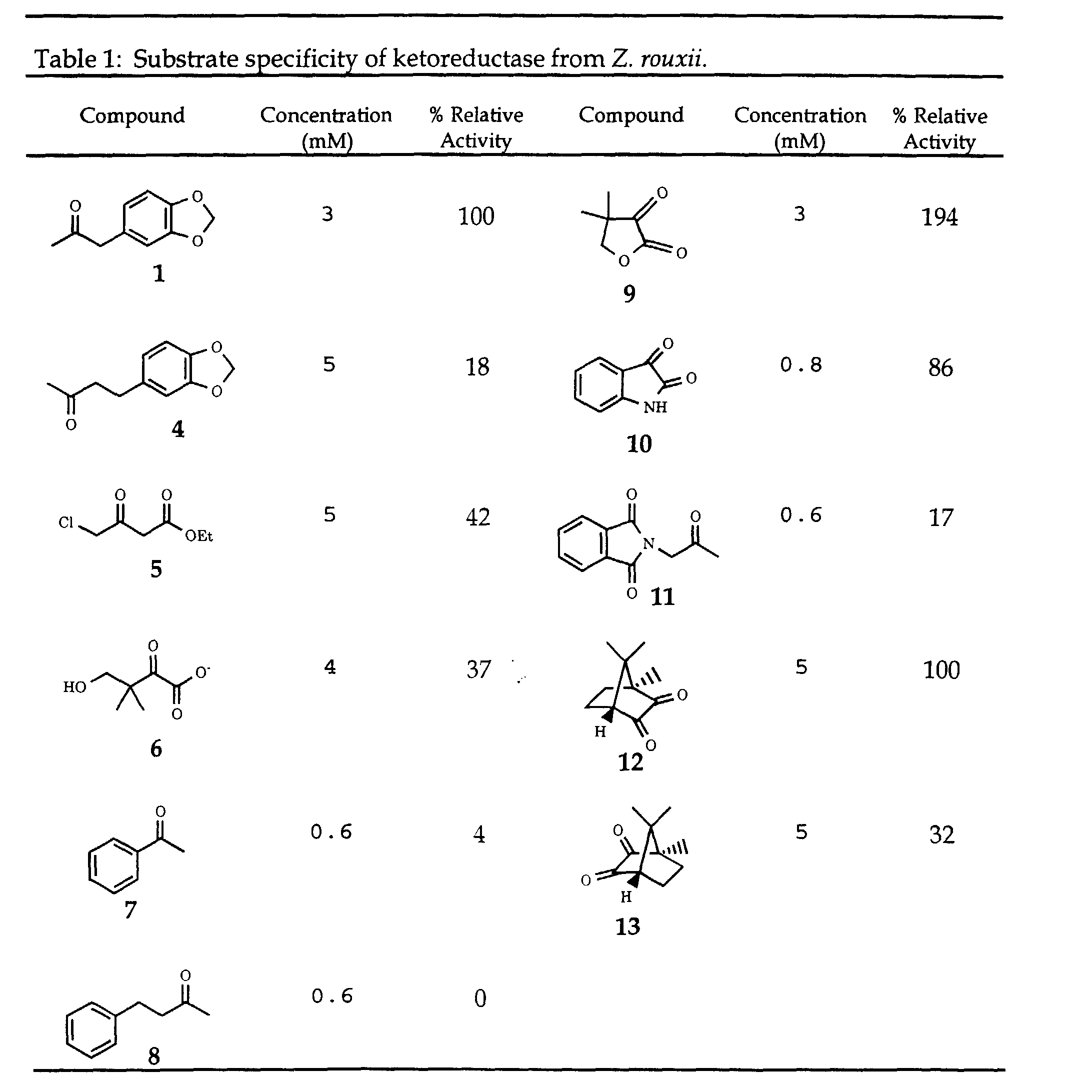

The ketoreductase enzymes disclosed herein are members of the carbonyl reductase enzyme class. Carbonyl reductases are involved in the reduction of xenobiotic carbonyl compounds (Hara *et. al, Arch. Biochem. Biophys.,* 244, 238-247, 1986) and have been classified into the short-chain dehydrogenase/reductase (SDR) enzyme superfamily (Jörnvall *et. al, Biochemistry,* 34, 6003-6013, 1995) and the single-domain reductase/epimerase/dehydrogenase (RED) enzyme superfamily (Labesse *et. al, Biochem. J.,* 304, 95-99, 1994). The ketoreductases of this invention are able to effectively reduce a variety of α-ketolactones, α-ketolactams, and diketones (Table 1).

The ketoreductase gene of *Z. rouxii* comprises a DNA sequence designated herein as SEQ ID NO:1. Those skilled in the art will recognize that owing to the degeneracy of the genetic code (i.e. 64 codons which encode 20 amino acids), numerous "silent" substitutions of nucleotide base pairs could be introduced into the sequence identified as SEQ ID NO:1 without altering the identity of the encoded amino acid(s) or protein product. All such substitutions are intended to be within the scope of the invention.

### Gene Isolation Procedures

Those skilled in the art will recognize that the ketoreductase gene may be obtained by a plurality of applicable recombinant DNA techniques including, for example, polymerase chain reaction (PCR) amplification, hybridization to a genomic or cDNA library, or de novo DNA synthesis. (*See e.g.,* J.Sambrook *et al.* Molecular Cloning, 2d Ed. Chap. 14 (1989)).

Methods for constructing cDNA libraries in a suitable vector such as a plasmid or phage for propagation in procaryotic or eucaryotic cells are well known to those skilled in the art. [*See e.g.* J.Sambrook *et al. Supra*]. Suitable cloning vectors are widely available.

Skilled artisans will recognize that the ketoreductase gene or fragment thereof could be isolated by PCR amplification from a human cDNA library prepared from a tissue in which said gene is expressed, using oligonucleotide primers targeted to any suitable region of SEQ ID NO:1. Methods for PCR amplification are widely known in the art. *See e.g.* PCR Protocols: A Guide to Method and Application, Ed. M. Innis *et.al.,* Academic Press (1990). The amplification reaction comprises template DNA, suitable enzymes, primers, nucleoside triphosphates, and buffers, and is conveniently carried out in a DNA Thermal Cycler (Perkin Elmer Cetus, Norwalk, CT). A positive result is determined by detecting an appropriately-sized DNA fragment following gel electrophoresis.

### Protein Production Methods

One embodiment of the present invention relates to the substantially purified ketoreductase enzyme (identified herein as SEQ ID NO:2) encoded by the *Z. rouxii* ketoreductase gene (identified herein as SEQ ID NO:1).

Skilled artisans will recognize that the proteins of the present invention can be synthesized by a number of different methods, such as chemical methods well known in the art, including solid phase peptide synthesis or recombinant methods. Both methods are described in U.S. Patent 4,617,149, incorporated herein by reference. The proteins of the invention can also be purified by well known methods from a culture of cells that produce the protein, for example, *Z. rouxii.*

The principles of solid phase chemical synthesis of polypeptides are well known in the art and may be found in general texts in the area. *See, e.g.,* H. Dugas and C. Penney, Bioorganic Chemistry (1981) Springer-Verlag, New York, 54-92. For example, peptides may be synthesized by solid-phase methodology utilizing an Applied Biosystems 430A peptide synthesizer (Applied Biosystems, Foster City, CA) and synthesis cycles supplied by Applied Biosystems.

The protein of the present invention can also be produced by recombinant DNA methods using the cloned ketoreductase gene. Recombinant methods are preferred if a high yield is desired. Expression of the cloned gene can be carried out in a variety of suitable host cells, well known to those skilled in the art. For this purpose, the ketoreductase gene is introduced into a host cell by any suitable means, well known to those skilled in the art. While chromosomal integration of the cloned gene is within the scope of the present invention, it is preferred that the gene be cloned into a suitable extra-chromosomally maintained expression vector so that the coding region of the ketoreductase gene is operably-linked to a constitutive or inducible promoter.

The basic steps in the recombinant production of the ketoreductase protein are:
a) constructing a natural, synthetic or semi-synthetic DNA encoding ketoreductase protein;
b) integrating said DNA into an expression vector in a manner suitable for expressing the ketoreductase protein, either alone or as a fusion protein; or integrating said DNA into a host chromosome such that said DNA expresses ketoreductase;
c) transforming or otherwise introducing said vector into an appropriate eucaryotic or prokaryotic host cell forming a recombinant host cell,
d) culturing said recombinant host cell in a manner to express the ketoreductase protein; and
e) recovering and substantially purifying the ketoreductase protein by any suitable means, well known to those skilled in the art.

### Expressing Recombinant ketoreductase Protein in Procaryotic and Eucaryotic Host Cells

Procaryotes may be employed in the production of the ketoreductase protein. For example, the *Escherichia coli* K12 strain 294 (ATCC No. 31446) or strain RV308 is particularly useful for the prokaryotic expression of foreign proteins. Other strains of *E. coli,* bacilli such as *Bacillus subtilis,* enterobacteriaceae such as *Salmonella typhimurium* or *Serratia marcescans,* various Pseudomonas species and other bacteria, such as *Streptomyces,* may also be employed as host cells in the cloning and expression of the recombinant proteins of this invention.

Promoter sequences suitable for driving the expression of genes in procaryotes include β-lactamase [*e.g.* vector pGX2907, ATCC 39344, contains a replicon and β -lactamase gene], lactose systems [Chang et al., Nature (London), 275:615 (1978); Goeddel et al., Nature (London), 281:544 (1979)], alkaline phosphatase, and the tryptophan (trp) promoter system [vector pATH1 (ATCC 37695) which is designed to facilitate expression of an open reading frame as a trpE fusion protein under the control of the trp promoter]. Hybrid promoters such as the tac promoter (isolatable from plasmid pDR540, ATCC-37282) are also suitable. Still other bacterial promoters, whose nucleotide sequences are generally known, enable one of skill in the art to ligate such promoter sequences to DNA encoding the proteins of the instant invention using linkers or adapters to supply any required restriction sites. Promoters for use in bacterial systems also will contain a Shine-Dalgarno sequence operably linked to the DNA encoding the desired polypeptides. These examples are illustrative rather than limiting.

The protein(s) of this invention may be synthesized either by direct expression or as a fusion protein comprising the protein of interest as a translational fusion with another protein or peptide which may be removable by enzymatic or chemical cleavage. It is often observed in the production of certain peptides in recombinant systems that expression as a fusion protein prolongs the lifespan, increases the yield of the desired peptide, or provides a convenient means of purifying the protein. A variety of peptidases (e.g. enterokinase and thrombin) which cleave a polypeptide at specific sites or digest the peptides from the amino or carboxy termini (e.g. diaminopeptidase) of the peptide chain are known. Furthermore, particular chemicals (e.g. cyanogen bromide) will cleave a polypeptide chain at specific sites. The skilled artisan will appreciate the modifications necessary to the amino acid sequence (and synthetic or semi-synthetic coding sequence if recombinant means are employed) to incorporate site-specific internal cleavage sites. *See e.g.,* P. Carter, "Site Specific Proteolysis of Fusion Proteins", Chapter 13, in Protein Purification: From Molecular Mechanisms to Large Scale Processes, American Chemical Society, Washington, D.C. (1990).

In addition to procaryotes, a variety of eucaryotic microorganisms including yeast are suitable host cells. The yeast *Saccharomyces cerevisiae* is the most commonly used eucaryotic microorganism. Other yeasts such as *Kluyveromyces lactis, Schizosaccharomyces pombe, and Pichia pastoris* are also suitable. For expression in *Saccharomyces,* the plasmid YRp7 (ATCC-40053), for example, may be used. *See, e.g.,* L. Stinchcomb, *et al., Nature,* 282:39 (1979); J. Kingsman *et al., Gene,* 7:141 (1979); *S.* Tschemper *et.al., Gene,* 10:157 (1980). Plasmid YRp7 contains the TRP1 gene which provides a selectable marker for use in a trpl auxotrophic mutant.

### Purification of Recombinantly-Produced ketoreductase Protein

An expression vector carrying a cloned ketoreductase gene is transformed or transfected into a suitable host cell using standard methods. Host cells may comprise procaryotes, such as *E. coli,* or simple eucaryotes, such as Z. *rouxii, S. cerevisiae, S. pombe, P. pastoris,* and *K. Lactis.* Cells which contain the vector are propagated under conditions suitable for expression of an encoded ketoreductase protein. If the recombinant gene has been placed under the control of an inducible promoter then suitable growth conditions would incorporate the appropriate inducer. The recombinantly-produced protein may be purified from cellular extracts of transformed cells by any suitable means.

In a preferred process for protein purification, the ketoreductase gene is modified at the 5' end to incorporate several histidine residues at the amino terminus of the ketoreductase protein product. This "histidine tag" enables a single-step protein purification method referred to as "immobilized metal ion affinity chromatography" (IMAC), essentially as described in U.S. Patent 4,569,794 which hereby is incorporated by reference. The IMAC method enables rapid isolation of substantially pure ketoreductase protein starting from a crude cellular extract.

Other embodiments of the present invention comprise isolated nucleic acid sequences which encode SEQ ID NO:2. As skilled artisans will recognize, the amino acid compounds of the invention can be encoded by a multitude of different nucleic acid sequences because most of the amino acids are encoded by more than one codon. Because these alternative nucleic acid sequences would encode the same amino acid sequences, the present invention further comprises these alternate nucleic acid sequences.

The ketoreductase genes disclosed herein, for example SEQ ID NO:1, may be produced using synthetic methodology. The synthesis of nucleic acids is well known in the art. *See, e.g.,* E.L. Brown, R. Belagaje, M.J. Ryan, and H.G. Khorana, Methods in Enzymology, 68:109-151 (1979). A DNA segment corresponding to a ketoreductase gene could be generated using a conventional DNA synthesizing apparatus, such as the Applied Biosystems Model 380A or 380B DNA synthesizers (Applied Biosystems, Inc., 850 Lincoln Center Drive, Foster City, CA 94404) which employ phosphoramidite chemistry. Alternatively, phosphotriester chemistry may be employed to synthesize the nucleic acids of this invention. [*See, e.g.,* M.J. Gait, ed., Oligonucleotide Synthesis, A Practical Approach, (1984).]

In an alternative methodology, namely PCR, a DNA sequence comprising a portion or all of SEQ ID NO:1, SEQ ID NO:4, SEQ ID NO:7, SEQ ID NO:10, or SEQ ID NO:13 can be generated from a suitable DNA source, for example *Z. rouxii* or *S. cerevisiae* genomic DNA or cDNA. For this purpose, suitable oligonucleotide primers targeting SEQ ID NO:1, SEQ ID NO:4, SEQ ID NO:7, SEQ ID NO:10, SEQ ID NO:13 or region therein are prepared, as described in U.S. Patent No. 4,889,818, which hereby is incorporated by reference. Protocols for performing the PCR are disclosed in, for example, PCR Protocols: A Guide to Method and Applications, Ed. Michael A. Innis *et al*., Academic Press, Inc. (1990).

The ribonucleic acids of the present invention may be prepared using the polynucleotide synthetic methods discussed *supra,* or they may be prepared enzymatically using RNA polymerase to transcribe a ketoreductase DNA template. *See e.g.,* J. Sambrook, *et. al., supra,* at 18.82-18.84.

This invention also provides nucleic acids, RNA or DNA, which are complementary to SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, or SEQ ID NO:15.

The present invention also provides probes and primers useful for a variety of molecular biology techniques including, for example, hybridization screens of genomic, subgenomic, or cDNA libraries. A nucleic acid compound comprising SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, or SEQ ID NO:15, or a complementary sequence thereof, or a fragment thereof, which is at least 18 base pairs in length, and which will selectively hybridize to DNA encoding a ketoreductase, is provided. Preferably, the 18 or more base pair compound is DNA. *See e.g.* B. Wallace and G. Miyada, "Oligonucleotide Probes for the Screening of Recombinant DNA Libraries," In Methods in Enzymology, Vol. 152, 432-442, Academic Press (1987).

Probes and primers can be prepared by enzymatic methods well known to those skilled in the art (*See e.g.* Sambrook *et al. supra*). In a most preferred embodiment these probes and primers are synthesized using chemical means as described above.

Another aspect of the present invention relates to recombinant DNA cloning vectors and expression vectors comprising the nucleic acids of the present invention. The preferred nucleic acid vectors are those which comprise DNA. The most preferred recombinant DNA vectors comprise a isolated DNA sequence selected from the group consisting of SEQ ID NO:1, SEQ ID NO:4, SEQ ID NO:7, SEQ ID NO:10, or SEQ ID NO:13.

The skilled artisan understands that choosing the most appropriate cloning vector or expression vector depends upon a number of factors including the availability of restriction enzyme sites, the type of host cell into which the vector is to be transfected or transformed, the purpose of the transfection or transformation (e.g., stable transformation as an extrachromosomal element, or integration into the host chromosome), the presence or absence of readily assayable or selectable markers (e.g., antibiotic resistance and metabolic markers of one type and another), and the number of copies of the gene to be present in the host cell.

Vectors suitable to carry the nucleic acids of the present invention comprise RNA viruses, DNA viruses, lytic bacteriophages, lysogenic bacteriophages, stable bacteriophages, plasmids, viroids, and the like. The most preferred vectors are plasmids.

When preparing an expression vector the skilled artisan understands that there are many variables to be considered, for example, whether to use a constitutive or inducible promoter. Inducible promoters are preferred because they enable high level, regulatable expression of an operably-linked gene. Constitutive promoters are further suitable in instances for which secretion or extra-cellular export is desireable. The skilled artisan will recognize a number of inducible promoters which respond to a variety of inducers, for example, carbon source, metal ions, and heat. The practitioner also understands that the amount of nucleic acid or protein to be produced dictates, in part, the selection of the expression system. The addition of certain nucleotide sequences is useful for directing the localization of a recombinant protein. For example, a sequence encoding a signal peptide preceding the coding region of a gene, is useful for directing the extra-cellular export of a resulting polypeptide.

Host cells harboring the nucleic acids disclosed herein are also provided by the present invention. Suitable host cells include procaryotes, such as *E. coli,* or simple eucaryotes, such as fungal cells, which have been transfected or transformed with a vector which comprises a nucleic acid of the present invention.

The present invention also provides a method for constructing a recombinant host cell capable of expressing SEQ ID NO:2, SEQ ID NO:5, SEQ ID NO:8, SEQ ID NO:11, or SEQ ID NO:14, said method comprising transforming or otherwise introducing into a host cell a recombinant DNA vector that comprises an isolated DNA sequence which encodes SEQ ID NO:2, SEQ ID NO:5, SEQ ID NO:8, SEQ ID NO:11, or SEQ ID NO:14. Preferred vectors for expression are those which comprise SEQ ID NO:1. Transformed host cells may be cultured under conditions well known to skilled artisans such that SEQ ID NO:2, SEQ ID NO:5, SEQ ID NO:8, SEQ ID NO:11, or SEQ ID NO:14 is expressed, thereby producing a ketoreductase protein in the recombinant host cell.

For the purpose of identifying or developing inhibitors or other modifiers of the enzymes disclosed herein, or for identifying suitable substrates for bioconversion, it would be desirable to identify compounds that bind and/or inhibit, or otherwise modify, the ketoreductase enzyme and its associated activity. A method for determining agents that will modify the ketoreductase activity comprises contacting the ketoreductase protein with a test compound and monitoring the alteration of enzyme activity by any suitable means.

The instant invention provides such a screening system useful for discovering compounds which bind the ketoreductase protein, said screening system comprising the steps of:
a) preparing ketoreductase protein;
b) exposing said ketoreductase protein to a test compound;
c) quantifying a modulation of activity by said compound.

Utilization of the screening system described above provides a means to determine compounds which may alter the activity of ketoreductase. This screening method may be adapted to automated procedures such as a PANDEX® (Baxter-Dade Diagnostics) system, allowing for efficient high-volume screening of potential modifying agents.

In such a screening protocol, ketoreductase is prepared as described herein, preferably using recombinant DNA technology. A test compound is introduced into a reaction vessel containing ketoreductase, followed by addition of enzyme substrate. For convenience the reaction can be coupled to the oxidation of NADPH, thereby enabling progress to be monitored spectrophotometrically by measuring the absorbance at 340 nm. Alternatively, substrate may be added simultaneously with a test compound. In one method radioactively or chemically-labeled compound may be used. The products of the enzymatic reaction are assayed for the chemical label or radioactivity by any suitable means. The absence or diminution of the chemical label or radioactivity indicates the degree to which the reaction is inhibited.

The following examples more fully describe the present invention. Those skilled in the art will recognize that the particular reagents, equipment, and procedures described are merely illustrative and are not intended to limit the present invention in any manner.

### EXAMPLE 1

### Construction of a DNA Vector for Expressing a Ketoreductase Gene in a Homologous or Heterologous Host

A plasmid comprising the *Z. rouxii* ketoreductase gene suitable for expressing said gene in a host cell, for example *E. coli* (DE3) strains, contains an origin of replication (Ori), an ampicillin resistance gene (Amp), useful for selecting cells which have incorporated the vector following a tranformation procedure, and further comprises the lacI gene for repression of the lac operon, as well as the T7 promoter and T7 terminator sequences in operable linkage to the coding region of the ketoreductase gene. Parent plasmid pET11A (obtained from Novogen, Madison, WI) was linearized by digestion with endonucleases *Nde*I and *BamHI.* Linearized pET11A was ligated to a DNA fragment bearing *Nde*I and *BamHI* sticky ends and further comprising the coding region of the *Z.rouxii* ketoreductase gene.

The ketoreductase gene is isolated most conveniently by the PCR. Genomic DNA from *Z. rouxii* isolated by standard methods was used for amplification of the ketoreductase gene. Primers are synthesized corresponding to the 5' and 3' ends of the gene (SEQ ID NO:1) to enable amplification of the coding region.

The ketoreductase gene (nucleotides 164 through 1177 of SEQ ID NO:1) ligated into the vector was modified at the 5' end (amino terminus of encoded protein) in order to simplify purification of the encoded ketoreductase protein. For this purpose, an oligonucleotide encoding 8 histidine residues and a factor Xa cleavage site was inserted after the ATG start codon at nucleotide positions 164 to 166 of SEQ ID NO:1. Placement of the histidine residues at the amino terminus of the encoded protein does not affect its activity and serves only to enable the IMAC one-step protein purification procedure.

### EXAMPLE 2

### Purification of Ketoreductase from Z. rouxii

Approximately 1 gram of *Z. rouxii* cell paste was resuspended in Lysing Buffer, comprising 50 mM Tris-Cl pH 7.5, 2 mM EDTA supplemented with pepstatin (1 µg/mL), leupeptin (1.25 µg/mL), aprotinin (2.5 µg/mL), and AEBSF (25 µg/mL). The cells were lysed using a DynoMill (GlenMills, Inc. Clifton, NJ) equipped with 0.5-0.75 mm lead free beads under continuous flow conditions according to the manufacturer's recommended use. After four complete passes through the DynoMill, the material was centrifuged twice (25,000 x *g* for 30 minutes at 4°C). Solid ammonium sulfate (291 g/liter) was added slowly to the resulting clarified cell extract with stirring at 4°C to achieve 50% saturation. After 1 hour, the mixture was centrifuged at 23,000 x *g* for 30 minutes. The supernatant was then brought to 85% saturation by the addition of solid ammonium sulfate (159 g/liter) and stirred for 1h at 4°C before centrifugation (23,000 *xg* for 30 min). The resultant 50-85% ammonium sulfate pellet was resuspended in 600 mL of Lysing Buffer and the residual ammonium sulfate was removed by dialysis against the same buffer at 4°C. The desalted material was centrifuged twice to remove particulate matter (23,000 *xg* for 30 min) and 700 - 800 Units of the clarified material was loaded onto a Red-120 dye affinity column (32 mm X 140 mm) equilibrated in 50 mM Tris-Cl pH 7.5, 1 mM MgCl₂, pepstatin (1 µg/mL), leupeptin (1.25 µg/mL), and aprotinin (2.5 µg/mL). Reductase activity was eluted from the column at a flowrate of 8 mL/min under the following conditions: 1) a 10 minute linear gradient from 0 - 0.3 M NaCl; 2) 13 minutes at 0.3 M NaCl; 3) a 60 minute linear gradient from 0.3 - 1.5 M NaCl. The fractions containing reductase activity were pooled, and changed to 20 mM potassium phosphate buffer (pH 7.2), pepstatin (1 µg/mL), leupeptin (1.25 µg/mL), and aprotinin (2.5 µg/mL) by dialysis at 4°C. The sample was clarified by centrifugation (23,000 x g for 30 min) and 400 Units was loaded onto a Bio-Scale CHT-I hydroxyapatite column (15 mm x 113 mm, Bio-Rad, Inc.) equilibrated in the same buffer that had been made 5% in glycerol. Reductase activity was eluted from the column at a flowrate of 5.0 mL/min in a sodium chloride step gradient consisting of 5 minutes at 0 M NaCl, a gradient step to 0.7 M NaCl which was maintained for 10 minutes, and then a 20 minute linear gradient from 0.7 - 1.0 M NaCl. The fractions containing reductase activity were pooled and desalted with 20 mM potassium phosphate buffer (pH 7.2), pepstatin A (1 µg/mL), leupeptin (1.25 µg/mL), and aprotinin (2.5 µg/mL) by dialysis at 4°C. The sample (100- 200 Units) was loaded onto a Bio-Scale CHT-I hydroxyapatite column (10 mm x 64 mm) equilibrated in the same buffer which had been made 5% in glycerol. Reductase activity was eluted from the column at a flowrate of 2.0 mL/min in a 25 minute linear gradient from 0 to 50% 400 mM potassium phosphate (pH 6.8), 5% glycerol. Fractions containing reductase activity were pooled and changed into 10 mM Tris-Cl (pH 8.5) by dialysis at 4°C. The sample was then made 10% in glycerol, concentrated to 0.4 mg/mL by ultrafiltration (Amicon, YM-10), and stored at-70°C.

### EXAMPLE 3

### Reductase Activity Using the Ketoreductase from Z. rouxii

Reductase activity was measured using a suitable substrate and a partially purified or substantially purified ketoreductase from *Z. rouxii.* Activity was measured as a function of the absorbance change at 340 nm, resulting from the oxidation of NADPH. The 1 ml assay contained a mixture of 3.0 mM 3,4-methylenedioxyphenyl acetone, 162 µM NADPH, 50 mM MOPS buffer (pH 6.8), and 0.6 mU of ketoreductase and was carried out at 26° C. Reaction mixtures were first equilibrated at 26°C for 10 min in the absence of NADPH, and then initiated by addition of NADPH. The absorbance was measured at 340 nm every 15 seconds over a 5 minute period; the change in absorbance was found to be linear over that time period. The kinetic parameters for 3,4-methylenedioxyphenyl acetone were determined at an NADPH concentration of 112 µM and a 3,4-methylenedioxyphenyl acetone concentration that varied from 1.7 mM - 7.2 mM. The kinetic parameters for NADPH were determined by maintaining the 3,4-methylenedioxyphenyl acetone concentration at 3 mM and the NADPH concentration was varied from 20.5 µM - 236.0 µM. An extinction coefficient of 6220 M⁻¹ cm⁻¹ for NADPH absorbance at 340 nm was used to calculate the specific activity of the enzyme. For assays using isatin, the change in absorbance with time was measured at 414 nm using an extinction coefficient of 849 M⁻¹ cm⁻¹ to calculate activity. One Unit of activity corresponds to 1 µmol of NADPH consumed per minute. For assays carried out at differing pH values, 10 mM Bis-Tris and 10 mM Tris were adjusted to the appropriate pH with HCl. Kinetic parameters were determined by non-linear regression using the JMP® statistics and graphics program.

### EXAMPLE 4

### Whole Cell Method for Stereoselective Reduction of Ketone Using Recombinant Yeast Cell

A vector for expressing the cloned *Z. rouxii* ketoreductase gene (SEQ ID NO:1) in a procaryotic or fungal cell, such as *S. cerevisiae,* is constructed as follows. A 1014 base pair fragment of *Z rouxii* genomic DNA or cDNA, carrying the ketoreductase gene, is amplified by PCR using primers targeted to the ends of the coding region specified in SEQ ID NO:1. It is desireable that the primers also incorporate suitable cloning sites for cloning of said 1014 base pair fragment into an expression vector. The appropriate fragment encoding ketoreductase is amplified and purified using standard methods, for cloning into an expression vector.

A suitable vector for expression in *E. coli* and *S. cerevisiae* is pYX213 (available from Novagen, Inc., 597 Science Drive, Madison, WI 53711; Code MBV-029-10), a 7.5 Kb plasmid that carries the following genetic markers: ori, 2µ circle, Amp^{R}, CEN, URA3, and the GAL promoter, for high level expression in yeast. Downstream of the GAL promoter, pYX213 carries a multiple cloning site (MCS), which will accommodate the ketoreductase gene amplified in the preceding step. A recombinant plasmid is created by digesting pYX213 and the amplified ketoreductase gene with a restriction enzyme, such as BamHl, and ligating the fragments together.

A recombinant expression vector carrying the *Z.rouxii* ketoreductase gene is transformed into a suitable Ura⁻ strain of *S. cerevisiae,* using well known methods. Ura⁺ transformants are selected on minimal medium lacking uracil.

Expression of the recombinant ketoreductase gene may be induced if desired by growing transformants in minimal medium that contains 2% galactose as the sole carbon source.

To carry out a whole cell stereospecific reduction, 3,4-methylenedioxyphenyl acetone is added to a culture of transformants to a concentration of about 10 grams per liter of culture. The culture is incubated with shaking at room temperature for 24 hours, and the presence of the chiral alcohol analyzed by HPLC.

## Claims

1. A substantially pure ketoreductase protein having the amino acid sequence which is SEQ ID NO:2.

2. An isolated nucleic acid compound encoding the protein of Claim 1, said protein having the amino acid sequence which is SEQ ID NO:2.

3. An isolated nucleic acid compound encoding the protein of Claim 1, wherein said compound has a sequence selected from the group consisting of:
(a) SEQ ID NO:1; or
(b) SEQ ID NO:3.

4. An isolated nucleic acid compound of Claim 3 wherein the sequence of said compound is SEQ ID NO:1

5. An isolated nucleic acid compound having a sequence complementary to SEQ ID NO:1.

6. An isolated nucleic acid compound of Claim 3 wherein the sequence of said compound is SEQ ID NO:3.

7. An isolated nucleic acid compound having a sequence complementary to SEQ ID NO:3.

8. A vector comprising an isolated nucleic acid compound of Claim 2.

9. A vector comprising an isolated nucleic.acid compound of Claim 3.

10. A vector of Claim 9, wherein said isolated nucleic acid compound is SEQ ID NO:1 operably-linked to a promoter sequence.

11. A host cell containing the vector of Claim 10.

12. A method for constructing a recombinant host cell having the potential to express SEQ ID NO:2, said method comprising introducing into said host cell by any suitable means a vector of Claim 9.

13. A method for expressing SEQ ID NO:2 in the recombinant host cell of Claim 12, said method comprising culturing said recombinant host cell under conditions suitable for gene expression.

14. A method for reducing a ketone in a stereospecific manner comprising providing a quantity of a suitable ketone to a culture of recombinant cells for a suitable period of time, wherein said cells are transformed with a vector that carries a ketoreductase gene, and wherein said cells express said ketoreductase gene.

15. A method, as in claim 14 wherein said gene is selected from the group consisting of SEQ ID NO:1, SEQ ID NO:4, SEQ ID NO:7, SEQ ID NO:10, and SEQ ID NO:13.

16. A method, as in claim 14 wherein said ketone comprises an α-ketolactone, α-ketolactam, or a diketone.

17. A method, as in Claim 14, wherein said recombinant cells are selected from the group consisting of *S. cerevisiae, Z. rouxii,* and *E. coli.*

18. A method for reducing a ketone in a stereospecific manner comprising mixing a quantity of a suitable ketone with a substantially purified ketoreductase and suitable reducing agent.

19. A method, as in Claim 18 wherein said ketoreductase is selected from the group consisting of SEQ ID NO:2, SEQ ID NO:5, SEQ ID NO:8, SEQ ID NO:11, and SEQ ID NO:14.

20. An isolated nucleic acid compound that encodes a protein having ketoreductase activity wherein said nucleic acid hybridizes under high stringency conditions to SEQ ID NO:1, SEQ ID NO:4, SEQ ID NO:7, SEQ ID NO:10, or SEQ ID NO:13.

21. A method, as in Claim 18 wherein said reducing agent is NADPH.
